(19) **Europäisches Patentamt / European Patent Office / Office européen des brevets**

(11) **EP 4 663 773 A1**

(12) # EUROPEAN PATENT APPLICATION

(43) Date of publication:
**17.12.2025 Bulletin 2025/51**

(21) Application number: **24382651.8**

(22) Date of filing: **14.06.2024**

(51) International Patent Classification (IPC):
***C12Q 1/6883*** (2018.01)

(52) Cooperative Patent Classification (CPC):
**C12Q 1/6883;** C12Q 2600/118; C12Q 2600/156;
C12Q 2600/172

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Patia Europe, S.L.
San Sebastián (ES)**

(72) Inventor: **The designation of the inventor has not
yet been filed**

(74) Representative: **Mewburn Ellis LLP
Aurora Building
Counterslip
Bristol BS1 6BX (GB)**

Remarks:
Claims 16-41 are deemed to be abandoned due to
non-payment of the claims fees (Rule 45(3) EPC).

(54) **METHODS, TOOLS AND SYSTEMS FOR THE SCREENING OF DIABETES STATUS**

(57) The present invention a method of predicting diabetes status in a subject, the method comprising determining the identity of at least one allele at each of at least four positions of single nucleotide polymorphism (SNP) selected from: rs371250843, rs9269173, rs17211699, rs2476601, rs10947332, rs7454108, rs9924471 and rs559242105, and/or an SNP in linkage disequilibrium with any one of said SNPs at $r^2 > 0.8$. Also provided are a genotyping tool, a diabetes classification system and type 1 diabetes risk assessment system for use in the method of the invention.

EP 4 663 773 A1

**Description**

*Field of the Invention*

The present invention relates to methods and tools, in particular arrays and related systems, for predicting diabetes status by assessing associated genetic variations.

*Background*

Diabetes is a chronic disease that causes a high level of glucose in the blood. The cause of this increase in glucose may be a lack of insulin or the body's resistance to it. Diabetes is currently one of the most common non-contagious diseases in the world, responsible for serious and numerous complications in the eyes, cardiovascular system, nervous system and urinary system. According to the 10th edition of the Diabetes Atlas of the International Diabetes Federation (IDF), in 2021, 537 million diabetic people between 20 and 79 years of age were registered worldwide, which corresponds to 1 in 10 people suffering from diabetes. Furthermore, this figure is expected to increase, reaching 643 million in 2030 and 783 million people with diabetes in 2045 (https://diabetesatlas.org/, accessed 3 June 2024).

Types of diabetes include Type 1 diabetes (T1D), Type 2 Diabetes (T2D), gestational diabetes and Monogenic diabetes (American Diabetes Association https://ada.com/conditions/diabetes/, accessed 3 June 2024). Of these, T1D and T2D are the most common.

Statistics show that 1 in 2 people with any type of diabetes are not diagnosed. This is equivalent to 240 million people with undetected diabetes in the world. If not treated properly, diabetes can cause severe complications in the patient, such as blindness, kidney disease, heart failure, lower limb amputations or stroke. Furthermore, among the diagnoses that are made, some types are incorrectly classified, which prevents these patients from receiving treatment appropriate to their needs. This situation is the cause of the IDF registering a total of 6.7 million deaths due to diabetes in the world, in addition to a health expenditure of 189 billion dollars. There is therefore a need for reliable methods of differentiating different forms of diabetes.

T2D is the most common form of diabetes and mostly occurs in adults. In T2D, the body no longer responds to insulin - this is termed insulin resistance. In turn, the body starts by producing more insulin, but then produces less over time (Goyal et al., 2024).

Monogenic diabetes results from a single genetic mutation (Goyal et al., 2024). Examples of monogenic diabetes include neonatal diabetes mellitus and maturity-onset diabetes of the young (MODY) (Goyal et al., 2024).

T1D is an autoimmune disorder characterised by the destruction of insulin producing β-cells, which leads to hyperglycaemia (Katsarou et al., 2017). It is a chronic condition, which requires careful maintenance of insulin levels. Complications of T1D include microvascular and macrovascular issues, neurocognitive changes as well as life threatening hypoglycaemia and ketoacidosis (Dimeglio et al., 2018). Ketoacidosis occurs when insulin levels reach such a low level that the body uses fat for energy rather than sugar. This releases ketones which cause blood to become acidic.

The burden of T D is vast. The prevalence of TID is predicted to increase from about 8.4 million individuals worldwide in 2021 to 13.5 - 17.4 million individuals by 2040 (Gregory et al., 2022). Although symptoms usually appear in children, they can develop at any age (Katsarou et al., 2017).

Before clinical symptoms such as loss of β-cell function, hyperglycaemia and ketoacidosis occur, autoantibodies can be detected in the patient's blood. As such, currently the risk of developing T1D is based upon detection of these autoantibodies. Targets of these autoantibodies include insulin, islet antigen-2 (IA-2), glutamic acid decarboxylase (GAD65) and zinc transporter-8 (ZnT8) (So et al 2021).

There are three main stages of progression of the disease as defined by the Eisenbarth model. The first stage is characterised by the presence of at least two autoantibodies, with normal blood glucose levels and no clinical symptoms. The second stage is characterised by the presence of at least two autoantibodies with changes in glucose metabolism and no clinical symptoms. The third stage is characterised by the onset of clinical symptoms (Primavera et al., 2020). The duration of each stage cannot be accurately predicted (Primavera et al., 2020. Furthermore, the stages are not always discrete and young children may bypass the second stage (Sims et al., 2022).

Having a relative with T1D increases the risk of an individual having the disease by 15-fold (Sims et al., 2022). For this reason, these individuals are the target of screening programmes and clinical trials. The TN10 trial demonstrated that a course of teplizumab was able to delay the clinical diagnosis of T1D in multiple autoantibody relatives without diabetes by a median of 24 months (Herold et al., 2019), highlighting the importance of early risk prediction of the disease. Teplizumab has since been approved for use in delaying onset of T1D by the FDA.

However, around 90% of individuals who are diagnosed with T1D have no family history of the disease (DiMeglio et al., 2018). These patients are therefore only screened once symptoms start to appear, by which time the opportunity to delay onset or to prepare for the onset has passed. Furthermore, delay of T1D would reduce the financial burden associated with the cost of treatments such as insulin and glucose monitoring (Sims et al., 2022). In many cases, individuals are diagnosed at the point of ketoacidosis. Importantly, early risk prediction and follow up monitoring of individuals identified as high risk would reduce the incidence of life threatening ketoacidosis at the time of diagnosis (Alonso et al., 2020). There is therefore a need for early risk prediction of T1D in the general population.

*Summary of the Invention*

Broadly, the present inventors have found that certain combinations of polymorphisms, particularly single nucleotide polymorphisms (SNPs), are associated with prediction of diabetes status. In particular, the methods can classify subjects into one of four categories: susceptibility to T1D, susceptibility to T2D, susceptibility to monogenic diabetes or no susceptibility to these forms of diabetes. The methods can also differentiate subjects at risk of T1D compared to control subjects or those with other forms of diabetes. Tools and associated systems have been developed for use in methods of the invention.

Accordingly, in a first aspect, the present invention provides a method of predicting diabetes status in a subject, the method comprising determining the identity of at least one allele at each of at least four positions of single nucleotide polymorphism (SNP) selected from:

> rs371250843;
> rs9269173;
> rs17211699;
> rs2476601;
> rs10947332;
> rs7454108;
> rs9924471; and
> rs559242105,

and/or an SNP in linkage disequilibrium with any one of said SNPs at $r^2 > 0.8$.

The method may further comprise determining the identity of at least one allele of at least one of the SNPs selected from:

> rs2524277;
> rs9268645;
> rs2187668;
> rs3842753; and
> rs4788084,

and/or an SNP in linkage disequilibrium with any one of said SNPs at $r^2 > 0.8$.

The predictive allele at each SNP may comprise:

> Del G at rs371250843;
> A at rs9269173;
> T at rs17211699;
> G at rs2476601;
> A at rs10947332;
> C at rs7454108;
> A at rs9924471;
> dupTA at rs559242105;
> A at rs2524277;
> G at rs9268645;
> T at rs2187668;
> T at rs3842753; and
> T at rs4788084.

In the method allele determination may be carried out at not more than 75 SNP positions.

The method may comprise determining the identity of both alleles at each SNP thereby obtaining the genotype of the subject at each SNP.

The method may comprise assaying a DNA-containing sample that has previously been obtained from said subject. Said sample may be selected from the group consisting of: blood, hair, skin, amniotic fluid, buccal swab, saliva, and faeces.

The method may comprise isolating and/or amplifying genomic DNA from said subject.

In some cases, determining the identity of said at least one allele at each SNP comprises: probe hybridization, real time PCR, array analysis, bead analysis, primer extension, restriction analysis and/or DNA sequencing.

Accurately differentiating between susceptibility to different forms of diabetes ensures the correct form of diabetes is diagnosed and the appropriate treatment and advice is given. Therefore, in some embodiments, predicting diabetes status comprises classifying the subject into one of the groups selected from susceptibility to T1D, susceptibility to T2D, susceptibility to monogenic diabetes and no susceptibility to diabetes.

In some embodiments, predicting diabetes status comprises classifying the subject into one of the groups selected from susceptibility to T1D, susceptibility to T2D and susceptibility to monogenic diabetes.

In some embodiments, the method comprises determining the identity of the genotype of the subject at each SNP and inputting the genotype identities into a probability function to compute said classification.

The probability function may comprises a logistic regression algorithm.

In some embodiments, predicting diabetes status comprises carrying out a computer-implemented multinomial logistic regression to identify the diabetes status for the sample i having the highest probability from the group of diabetes statuses consisting of: T1D, T2D, monogenic diabetes and no diabetes, according to the following formulae (1) and (2):

$$p(Y_i = k|X_i) = \frac{e^{\beta_k \cdot X_i}}{1+\sum_{j=1}^{K-1} e^{\beta_j \cdot X_i}}, k < K \qquad (1)$$

$$p(Y_i = K|X_i) = \frac{1}{1+\sum_{j=1}^{K-1} e^{\beta_j \cdot X_i}} \qquad (2)$$

wherein:

K represents the four outcomes T1D (k=1), T2D (k=2), monogenic diabetes (k=3) and no diabetes (k=4; the "pivot outcome"),

$\beta_k$ denotes the vector of learned regression coefficients for the $k^{th}$ outcome, said regression coefficients comprising a coefficient for each input feature and an intercept value,

X denotes the vector of m input features of sample i, wherein said input features comprise the number of predictive alleles at each SNP, $p(Y_i=k|X_i)$ for k < K denotes the probability that the sample i has the outcome $k$ for k=1, $k=2$ and k=3, $p(Y_i=K|X_i)$ denotes the probability that the sample i has the outcome k=4.

In some embodiments, predicting diabetes status comprises classifying the subject into one of the groups selected from susceptibility to T1D, susceptibility to T2D and susceptibility to monogenic diabetes.

In some embodiments, the method comprises determining the identity of the genotype of the subject at each SNP and inputting the genotype identities into a probability function to compute said classification.

The probability function may comprises a logistic regression algorithm.

In some embodiments, predicting diabetes status comprises carrying out a computer-implemented multinomial logistic regression to identify the diabetes status for the sample i having the highest probability from the group of diabetes statuses consisting of: T1D, T2D and monogenic diabetes, according to the following formulae (1) and (2):

$$p(Y_i = k|X_i) = \frac{e^{\beta_k \cdot X_i}}{1+\sum_{j=1}^{K-1} e^{\beta_j \cdot X_i}}, k < K \qquad (1)$$

$$p(Y_i = K|X_i) = \frac{1}{1+\sum_{j=1}^{K-1} e^{\beta_j \cdot X_i}} \qquad (2)$$

wherein:

K represents the three outcomes T1D (k=1), T2D (k=2), monogenic diabetes (k=3; the "pivot outcome"),

$\beta_k$ denotes the vector of learned regression coefficients for the $k^{th}$ outcome, said regression coefficients comprising a coefficient for each input feature and an intercept value,

X denotes the vector of m input features of sample i, wherein said input features comprise the number of predictive alleles at each SNP,

$p(Y_i=k|X_i)$ for k < K denotes the probability that the sample i has the outcome $k$ for k=1 and k=2, $p(Y_i=K|X_i)$ denotes the probability that the sample i has the outcome k=3.

In some embodiments, predicting diabetes status comprises assessing the probability that a subject will develop T1D compared to T2D, monogenic diabetes and/or no diabetes.

In some embodiments, predicting diabetes status comprises assessing the probability that a subject will develop T1D compared to T2D, monogenic diabetes and no diabetes.

In some embodiments, predicting diabetes status comprises assessing the probability that a subject will develop T1D compared to T2D.

In some embodiments, predicting diabetes status comprises assessing the probability that a subject will develop T1D compared to monogenic diabetes.

In some embodiments, the monogenic diabetes is MODY or neonatal diabetes mellitus. In preferred embodiments, the monogenic diabetes is MODY.

In some embodiments, the method comprises determining the identity of the genotype of the subject at each SNP, and wherein the method further comprises computing a T1D risk score for said subject based on the identity of the genotype at each SNP.

In some embodiments, the method comprises inputting the genotype identities into a probability function to compute said risk score. The probability function may comprise a logistic regression algorithm.

In some embodiments, computing the risk score from the genotype data comprises weighting the contribution of each SNP found to be present such that the contribution of each SNP to the risk score is commensurate with an odds ratio for the association of the SNP to type 1 diabetes, as set forth in Tables 8 or 10.

In some embodiments, computing said risk score comprises using the following formula:

$$risk\ score\ \ p = \frac{1}{1 + e^{-(-\beta_0 + \sum_{i=1}^{m} \beta_i x_i)}}$$

wherein $\beta_0$ is an intercept weight, $\beta$ is a vector of weights for each of m variables and x is a vector of variables associated with the subject, wherein the variables comprise the number of predictive alleles present at each SNP and optionally variables derived therefrom.

In a second aspect, the invention provides a system comprising:

a). a processor; and
b). a computer readable medium comprising instructions that, when executed by the processor, cause the processor to perform the methods described herein.

In a third aspect, the invention provides a non-transitory computer readable medium or media comprising instructions that, when executed by at least one processor, cause the at least one processor to perform the methods described herein.

In some embodiments, following identification of the at least one allele at each single nucleotide polymorphism (SNP), if the subject is determined to be at risk of developing Type 1 Diabetes, the method further comprises administering to the subject a test selected from the group consisting of: detection of type 1 diabetes autoantibodies, a glycated hemoglobin (A1C) test, a blood sugar test or a C-peptide test.

The detection of type 1 diabetes autoantibodies may comprise detection of islet cell autoantibodies, glutamic acid decarboxylase autoantibodies, insulin autoantibodies and/or protein tyrosine phosphatase autoantibodies.

In a fourth aspect, the invention provides a method of preventing or delaying onset of Type 1 Diabetes in a subject, the method comprising determining the identity of at least one allele at each of at least four positions of single nucleotide polymorphism (SNP) selected from:

rs371250843;
rs9269173;
rs17211699;
rs2476601;
rs10947332;
rs7454108;
rs9924471; and
rs559242105,

and/or an SNP in linkage disequilibrium with any one of said SNPs at $r^2 > 0.8$;

determining the subject to be at risk of developing T1D, and administering a medicament for preventing or delaying the onset of T1D.

The method may further comprises determining the identity of at least one allele of at least one of the SNPs selected from:

rs2524277;
rs9268645;
rs2187668;
rs3842753; and
rs4788084,

and/or an SNP in linkage disequilibrium with any one of said SNPs at $r^2 > 0.8$.

In some embodiments, the medicament is Teplizumab.

In a fifth aspect, the invention provides a genotyping tool for use in the methods described herein, said tool comprising an array having a plurality of oligonucleotide probe pairs, each of said probe pairs comprising a first probe specific for a first allele of a single nucleotide polymorphism (SNP) and a second probe specific for a second allele of the SNP, wherein said plurality of oligonucleotide probe pairs comprises probe pairs that interrogate at least four of the following SNPs:

rs371250843;
rs9269173;
rs17211699;
rs2476601;
rs10947332;
rs7454108;
rs9924471; and
rs559242105,

and/or an SNP in linkage disequilibrium with any one of said SNPs at $r^2 > 0.8$.

The genotyping tool may further comprising oligonucleotide probe pairs that interrogate at least one of the SNPs selected from:

rs2524277;
rs9268645;
rs2187668;
rs3842753; and
rs4788084,

and/or an SNP in linkage disequilibrium with any one of said SNPs at $r^2 > 0.8$.

In some embodiments, the oligonucleotide probes of the array that interrogate SNPs selected from rs371250843, rs9269173, rs17211699, rs2476601, rs10947332, rs7454108, rs9924471 and rs559242105 make up at least 50% of the probes present in the array.

In some embodiments, the total number of different SNPs for which allele-specific probes are provided does not exceed 25.

In some embodiments, the tool is in the form of a TaqMan® OpenArray® SNP genotyping platform, a Dynamic Array integrated fluidic circuits (IFC) genotyping platform, a next-generation sequencing system, or a MassARRAY® system.

In some embodiments, the allele-specific oligonucleotide probes are each covalently attached to a fluorophore.

In some embodiments, the tool further comprises a primer pair for each of said SNPs, said primer pair for each SNP comprising an oligonucleotide primer that hybridizes to a target sequence upstream of the SNP and an oligonucleotide primer that hybridizes to a target sequence downstream of the SNP.

In some embodiments, wherein the tool further comprises one or more reagents for amplification of DNA comprising said SNPs and/or for detection of said allele-specific probes.

In a sixth aspect, the invention provides a diabetes classification system for use in a method of the first aspect, the system comprising a genotyping tool as described herein and a computer programmed to classify a subject into one of the groups selected from susceptibility to T1D, susceptibility to T2D, susceptibility to monogenic diabetes and no susceptibility to diabetes from the genotype data of the subject at each of the SNPs set forth in the first aspect.

In a seventh aspect, the invention provides a diabetes classification system for use in a method of the first aspect, the system comprising a genotyping tool as described herein and a computer programmed to classify a subject into one of the groups selected from susceptibility to T1D, susceptibility to T2D and susceptibility to monogenic diabetes from the genotype data of the subject at each of the SNPs set forth in the first aspect.

In an eighth aspect, the invention provides a Type 1 Diabetes risk assessment system for use in a method as defined in the first aspect, the system comprising a genotyping tool as described herein and a computer programmed to compute a Type 1 Diabetes risk score from the genotype data of the subject at each of at the SNPs set forth in the first aspect. In this aspect, computing the risk score from the genotype data may comprise weighting the contribution of each SNP found to be present such that the contribution of each SNP to the risk score is commensurate with an odds ratio for the association of the SNP to type 1 diabetes, as set forth in Tables 8 or 10.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:

Figure 1. ROC curve for T1D based on multinomial algorithm
Figure 2: ROC curve for T2D based on multinomial algorithm
Figure 3: ROC curve for monogenic diabetes based on multinomial algorithm

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Single nucleotide polymorphisms (SNPs)

SNPs are identified herein using the rs identifier numbers in accordance with the NCBI dbSNP database, which is publically available at: https://www.ncbi.nlm.nih.gov/snp/. As used herein, rs numbers refer to the dbSNP Homo sapiens build 156 available from 21 September 2022.

### Linkage disequilibrium (LD)

In some embodiments, SNPs in linkage disequilibrium with the SNPs associated with the invention are useful for obtaining similar results. As used herein, linkage disequilibrium refers to the non-random association of SNPs at two or more loci. Techniques for the measurement of linkage disequilibrium are known in the art. As two SNPs are in linkage disequilibrium if they are inherited together, the information they provide is correlated to a certain extent. SNPs in linkage disequilibrium with the SNPs included in the models can be obtained from databases such as HapMap or other related databases, from experimental setups run in laboratories or from computer-aided in-silico experiments. Determining the genotype of a subject at a position of SNP as specified herein, e.g. as specified by NCBI dbSNP rs identifier, may comprise directly genotyping, e.g. by determining the identity of the nucleotide of each allele at the locus of SNP, and/or indirectly genotyping, e.g. by determining the identity of each allele at one or more loci that are in linkage disequilibrium with the SNP in question and which allow one to infer the identity of each allele at the locus of SNP in question with a substantial degree of confidence. In some cases, indirect genotyping may comprise determining the identity of each allele at one or more loci that are in sufficiently high linkage disequilibrium with the SNP in question so as to allow one to infer the identity of each allele at the locus of SNP in question with a probability of at least 90%, at least 95% or at least 99% certainty.

As will be appreciated by the reader, in some cases one or more polymorphisms or alterations in linkage disequilibrium with a polymorphism or alteration disclosed herein may find use the methods of the present invention. Linkage disequilibrium (LD) is a phenomenon in genetics whereby two or more mutations or polymorphisms are in such close genetic proximity that they are co-inherited. This means that in genotyping, detection of one polymorphism as present infers the presence of the other. Thus, a polymorphism or alteration in such linkage disequilibrium acts as a surrogate marker for a polymorphism or alteration as disclosed herein. Preferably, reference herein to a polymorphism or alteration in linkage disequilibrium with another means that $r2 > 0.8$, preferably $r2 > 0.9$, more preferably $r2 > 0.95$ or even $r2 > 0.99$. In particularly preferred embodiments, an SNP is considered to be in LD with an SNP set forth in Table 1 if it exhibits $r2 = 1.0$ and $D' = 1.0$.

In one example, the SNP rs559242105 is believed to be in strong LD with the SNP rs2281389, such that rs2281389 may in some cases be used, in accordance with any aspect or embodiment of the present invention, as a proxy SNP for rs559242105.

### *Predicting diabetes status*

In the present methods, the presence of one or more predictive alleles at a combination of SNPs is used to determine susceptibility to diabetes. The methods of the invention can also distinguish between different types of diabetes.

In a first aspect, the present invention provides a method of predicting diabetes status in a subject, the method comprising determining the identity of at least one allele at each of at least four positions (e.g. 4, 5, 6, 7 or 8) of single nucleotide polymorphism (SNP) selected from:

    rs371250843;
    rs9269173;
    rs17211699;
    rs2476601;
    rs10947332;
    rs7454108;
    rs9924471; and
    rs559242105,

and/or an SNP in linkage disequilibrium with any one of said SNPs at $r^2 > 0.8$, $r^2 > 0.9$, $r^2 > 0.95$, $r^2 > 0.99$ or $r^2 = 1.0$.

Any SNP in linkage disequilibrium with any one of the listed SNPs may be used as a proxy for the listed SNP. For example, the SNP rs559242105 is believed to be in strong linkage disequilibrium with the SNP rs2281389, such that rs2281389 may be used, in accordance with any aspect of the present invention, as a proxy SNP for rs559242105.

Any SNP in linkage disequilibrium with any one of the listed SNPs may be measured in addition to those SNPs listed.

The method may further comprises determining the identity of at least one allele of at least one (e.g. 1, 2, 3, 4 or 5) of the SNPs selected from:

    rs2524277;
    rs9268645;
    rs2187668;
    rs3842753; and
    rs4788084,

and/or an SNP in linkage disequilibrium with any one of said SNPs at $r^2 > 0.8$, $r^2 > 0.9$, $r^2 > 0.95$, $r^2 > 0.99$ or $r^2 = 1.0$.

The predictive allele at each SNP may comprise:

Del G at rs371250843;
A at rs9269173;
T at rs17211699;
G at rs2476601;
A at rs10947332;
C at rs7454108;
A at rs9924471;
dupTA at rs559242105;
A at rs2524277;
G at rs9268645;
T at rs2187668;
T at rs3842753; and
T at rs4788084.

The predictive allele represents that which is alternative to the reference genome on the SNP database, which is publicly available at https://www.ncbi.nlm.nih.gov/snp/. Table 1 shows the gene name and predictive allele associated with the SNPs described in the present disclosure.

In the methods, allele determination may be carried out at not more than 100, 90, 80, 75, 70, 60, 50, 40, 30, 25, 20, 15, 14, 13, 12, 11, 10, 9, 8, 7, 6, 5 or 4 SNP positions.

The methods may comprise determining the identity of both alleles at each SNP thereby obtaining the genotype of the subject at each SNP. For example, the methods may comprise determining the identity of both alleles at 4, 5, 6, 7, 8, 9, 10, 11, 12 or 13 of the above listed SNPs.

The methods may comprise assaying a DNA-containing sample that has previously been obtained from said subject. The sample may be selected from the group consisting of: blood, hair, skin, amniotic fluid, buccal swab, saliva, and feces.

The methods may comprise isolating and/or amplifying genomic DNA from said subject.

In the methods, determining the identity of said at least one allele at each SNP may comprise: probe hybridization, real time PCR, array analysis, bead analysis, primer extension, restriction analysis and/or DNA sequencing.

The subject described herein in may be any mammalian subject. Preferably the subject is human. The subject may be male or female. The subject may have a family history of diabetes (e.g. T1D or T2D). Alternatively, the subject may not have a family history of diabetes (e.g. T1D or T2D).

Table 1: Gene and predictive allele associated with each SNP.

| SNP | Gene | Predictive allele |
| --- | --- | --- |
| rs2476601 | PTPN22 | G |
| rs782810 | MGAT4FP | G |
| rs6742799 | RBMS1 | C |
| rs560887 | G6PC2 | C |
| rs1496653 | UBE2E2 | G |
| rs831571 | | C |
| rs4402960 | IGF2BP2 | T |
| rs7649121 | ADIPOQ | T |
| rs6808574 | LOC107986166 | C |
| rs10010131 | WFS1 | G |
| rs10517086 | LINC02357 | A |
| rs371250843 | HLA-B | GGGG (del G) |
| rs2524277 | LINC01149 | A |
| rs9268645 | HLA-DRA | G |
| rs9269173 | | A |

(continued)

| SNP | Gene | Predictive allele |
|---|---|---|
| rs17211699 | HLA-DQB1-AS1 | T |
| rs9273369 | HLA-DQB1-AS1 | C |
| rs10947332 | | A |
| rs7454108 | | C |
| rs559242105 | | TATATATATA (dupTA) |
| rs9470794 | ZFAND3 | C |
| rs4407733 | | G |
| rs4607517 | | A |
| rs3996352 | LOC105375508 | G |
| rs3802177 | SLC30A8 | A |
| rs1574285 | GLIS3 | T |
| rs7020673 | GLIS3 | G |
| rs2104286 | IL2RA | C |
| rs4746890 | | C |
| rs7903146 | TCF7L2 | T |
| rs2421016 | PLEKHA1 | T |
| rs689 | INS | T |
| rs7111341 | | T |
| rs231362 | KCNQ1OT1 | G |
| rs231361 | KCNQ1OT1 | A |
| rs5215 | KCNJ11 | T |
| rs7944584 | MADD | T |
| rs1552224 | ARAP1 | C |
| rs10219509 | LOC105369612 | C |
| rs7138803 | | A |
| rs4759229 | ERBB3 | G |
| rs653178 | ATXN2 | T |
| rs7957197 | OASL | A |
| rs9585056 | | T |
| rs2289702 | CTSH | T |
| rs8042680 | PRC1 | A |
| rs12899811 | VPS33B | G |
| rs12444268 | | A |
| rs9924471 | SGF29 | A |
| rs7202877 | | G |
| rs45450798 | PTPN2 | G |
| rs763361 | CD226 | C |
| rs10423928 | GIPR | A |
| rs5763779 | HORMAD2 | G |

(continued)

| SNP | Gene | Predictive allele |
|---|---|---|
| rs10401969 | SUGP1 | C |
| rs36608 | MTMR3 | G |
| rs17791513 | | G |
| rs16996148 | | T |
| rs429358 | APOE | C |
| rs10503669 | | A |
| rs1801282 | PPARG | G |
| rs10203174 | THADA | T |
| rs9921586 | | T |
| rs1531343 | RPSAP52 | C |
| rs10885122 | | G |
| rs10923931 | NOTCH2 | T |
| rs7607980 | COBLL1 | C |
| rs1106240 | PITPNM2 | T |
| rs17168486 | DGKB | T |
| rs1031605 | SCARB1 | A |
| rs35767 | IGF1 | G |
| rs10842994 | LOC105369709 | T |
| rs10811661 | | C |
| rs17265513 | ZHX3 | C |
| rs1182397 | UBE3C | G |
| rs10840595 | | T |
| rs553668 | ADRA2A | G |
| rs11920090 | SLC2A2 | A |
| rs4812829 | HNF4A-AS1 | A |
| rs10146997 | NRXN3 | G |
| rs61839660 | IL2RA | T |
| rs12189871 | | T |
| rs144530872 | | A |
| rs12251307 | | T |
| rs16956936 | DNAH2 | T |
| rs9259118 | LOC124905339 | C |
| rs72848653 | | T |
| rs3024505 | | A |
| rs425105 | PRKD2 | C |
| rs2187668 | HLA-DQA1 | T |
| rs11258747 | PRKCQ | T |
| rs2816316 | LOC105371664 | A |
| rs947474 | LINC02656 | A |

(continued)

| SNP | Gene | Predictive allele |
|---|---|---|
| rs72928038 | BACH2 | A |
| rs17574546 | | C |
| rs2327832 | | G |
| rs3842753 | INS-IGF2 | T |
| rs4788084 | | T |
| rs3184504 | SH2B3 | C |

*Classification of diabetes types*

The methods of the present invention can be used to classify subjects into one of several groups. For example, they can classify subjects into one of the four following groups: susceptibility to T1D, susceptibility to T2D, susceptibility to monogenic diabetes or no susceptibility to these forms of diabetes.

Accordingly, in some embodiments, predicting diabetes status comprises classifying the subject into one of the groups selected from susceptibility to T1D, susceptibility to T2D, susceptibility to monogenic diabetes and no susceptibility to diabetes. In some embodiments, the patient may already have developed diabetes and the methods described herein are used to differentiate between T1D, T2D, monogenic diabetes and no diabetes, and therefore aid in providing an accurate diagnosis.

In some embodiments, the invention provides a method of classifying a subject into one of the groups selected from susceptibility to T1D, susceptibility to T2D, susceptibility to monogenic diabetes and no susceptibility to diabetes, the method comprising determining the identity of at least one allele at each of at least four (e.g. 4, 5, 6, 7 or 8) of the single nucleotide polymorphisms (SNPs) selected from:

rs371250843;
rs9269173;
rs17211699;
rs2476601;
rs10947332;
rs7454108;
rs9924471; and
rs559242105,

and/or an SNP in linkage disequilibrium with any one of said SNPs at $r^2 > 0.8$, $r^2 > 0.9$, $r^2 > 0.95$, $r^2 > 0.99$ or $r^2 = 1.0$.

The method may further comprise determining the identity of at least one allele of at least 1 SNP referred to in Table 3. For example, the method may further comprise determining the identity of 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45 or all of at least one allele of the SNPs referred to in Table 3.

The predictive allele associated with each of these SNPs is defined in Table 1. The presence of the predictive allele may determine the classification of the subject. The gene may be null, homozygous or heterozygous for the predictive allele.

In some embodiments, the methods may comprise determining the identity of at least one allele (e.g. one or two) from the following SNPs, wherein the presence of one or more of the following predictive alleles determines the classification of the subject:

| | | |
|---|---|---|
| G | at | rs2476601; |
| G | at | rs782810 ; |
| C | at | rs6742799; |
| C | at | rs560887 ; |
| G | at | rs1496653; |
| C | at | rs831571; |
| T | at | rs4402960; |
| T | at | rs7649121; |

(continued)

| | | |
|---|---|---|
| C | at | rs6808574; |
| G | at | rs10010131; |
| A | at | rs10517086; |
| del G | at | rs371250843; |
| A | at | rs2524277; |
| G | at | rs9268645; |
| A | at | rs9269173; |
| T | at | rs17211699; |
| C | at | rs9273369; |
| A | at | rs10947332; |
| C | at | rs7454108; |
| dupTA | at | rs559242105; |
| C | at | rs9470794; |
| G | at | rs4407733; |
| A | at | rs4607517; |
| G | at | rs3996352; |
| A | at | rs3802177; |
| T | at | rs1574285; |
| G | at | rs7020673; |
| C | at | rs2104286; |
| C | at | rs4746890; |
| T | at | rs7903146; |
| T | at | rs2421016; |
| T | at | rs689; |
| T | at | rs7111341; |
| G | at | rs231362; |
| A | at | rs231361; |
| T | at | rs5215; |
| T | at | rs7944584; |
| C | at | rs1552224; |
| C | at | rs10219509; |
| A | at | rs7138803; |
| G | at | rs4759229; |
| T | at | rs653178; |
| A | at | rs7957197; |
| T | at | rs9585056; |
| T | at | rs2289702; |
| A | at | rs8042680; |
| G | at | rs12899811; |
| A | at | rs12444268; |
| A | at | rs9924471; |
| G | at | rs7202877; |
| G | at | rs45450798; |
| C | at | rs763361; |
| A | at | rs10423928; and |
| G | at | rs5763779. |

In some embodiments, the method comprises determining the identity of the genotype of the subject at each SNP and inputting the genotype identities into a probability function to compute said classification.

The probability function may comprises a logistic regression algorithm. Each SNP may be associated with a specific weighting and the genotype data from a combination of SNPs can be used to classify subjects. The logistic regression algorithm may also be based on other variables in addition to the SNPs, such as sex of the subject.

In some embodiments, predicting diabetes status comprises carrying out a computer-implemented multinomial logistic regression to identify the diabetes status for the sample i having the highest probability from the group of diabetes statuses consisting of: susceptibility to T1D, susceptibility to T2D, susceptibility to monogenic diabetes and no susceptibility to diabetes, according to the following formulae (1) and (2):

$$p(Y_i = k|X_i) = \frac{e^{\beta_k \cdot X_i}}{1 + \sum_{j=1}^{K-1} e^{\beta_j \cdot X_i}}, k < K \qquad (1)$$

$$p(Y_i = K|X_i) = \frac{1}{1 + \sum_{j=1}^{K-1} e^{\beta_j \cdot X_i}} \qquad (2)$$

wherein:

K represents the four outcomes susceptibility to T1D (k=1), susceptibility to T2D (k=2), susceptibility to monogenic diabetes (k=3) and no susceptibility to diabetes (k=4; the "pivot outcome"),

$\beta_k$ denotes the vector of learned regression coefficients for the $k^{th}$ outcome, said regression coefficients comprising a coefficient for each input feature and an intercept value,

X denotes the vector of m input features of sample i, wherein said input features comprise the number of predictive alleles at each of at least the following SNPs:

| | | |
|---|---|---|
| G | at | rs2476601; |
| G | at | rs782810 ; |
| C | at | rs6742799; |
| C | at | rs560887 ; |
| G | at | rs1496653; |
| C | at | rs831571; |
| T | at | rs4402960; |
| T | at | rs7649121; |
| C | at | rs6808574; |
| G | at | rs10010131; |
| A | at | rs10517086; |
| del G | at | rs371250843; |
| A | at | rs2524277; |
| G | at | rs9268645; |
| A | at | rs9269173; |
| T | at | rs17211699; |
| C | at | rs9273369; |
| A | at | rs10947332; |
| C | at | rs7454108; |
| dupTA | at | rs559242105; |
| C | at | rs9470794; |
| G | at | rs4407733; |
| A | at | rs4607517; |
| G | at | rs3996352; |
| A | at | rs3802177; |
| T | at | rs1574285; |
| G | at | rs7020673; |
| C | at | rs2104286; |
| C | at | rs4746890; |
| T | at | rs7903146; |
| T | at | rs2421016; |
| T | at | rs689; |
| T | at | rs7111341; |
| G | at | rs231362; |

(continued)

| | | |
|---|---|---|
| A | at | rs231361; |
| T | at | rs5215; |
| T | at | rs7944584; |
| C | at | rs1552224; |
| C | at | rs10219509; |
| A | at | rs7138803; |
| G | at | rs4759229; |
| T | at | rs653178; |
| A | at | rs7957197; |
| T | at | rs9585056; |
| T | at | rs2289702; |
| A | at | rs8042680; |
| G | at | rs12899811; |
| A | at | rs12444268; |
| A | at | rs9924471; |
| G | at | rs7202877; |
| G | at | rs45450798; |
| C | at | rs763361; |
| A | at | rs10423928; and |
| G | at | rs5763779, |

$p(Y_i=k|X_j)$ for k < K denotes the probability that the sample i has the outcome $k$ for k=1, $k=2$ and k=3, $p(Y_i=K|X_j)$ denotes the probability that the sample i has the outcome k=4.

In some embodiments, predicting diabetes status comprises carrying out a computer-implemented multinomial logistic regression to identify the diabetes status for the sample i having the highest probability from the group of diabetes statuses consisting of: T1D, T2D, monogenic diabetes and no diabetes, according to the following formulae (1) and (2):

$$p(Y_i = k|X_i) = \frac{e^{\beta_k \cdot X_i}}{1+\sum_{j=1}^{K-1} e^{\beta_j \cdot X_i}}, k < K \qquad (1)$$

$$p(Y_i = K|X_i) = \frac{1}{1+\sum_{j=1}^{K-1} e^{\beta_j \cdot X_i}} \qquad (2)$$

wherein:

K represents the four outcomes T1D (k=1), T2D (k=2), monogenic diabetes (k=3) and no diabetes (k=4; the "pivot outcome"),

$\beta_k$ denotes the vector of learned regression coefficients for the kth outcome, said regression coefficients comprising a coefficient for each input feature and an intercept value,

X denotes the vector of m input features of sample i, wherein said input features comprise the number of predictive alleles at each of at least the following SNPs:

| | | |
|---|---|---|
| G | at | rs2476601; |
| G | at | rs782810 ; |
| C | at | rs6742799; |
| C | at | rs560887 ; |
| G | at | rs1496653; |
| C | at | rs831571; |
| T | at | rs4402960; |
| T | at | rs7649121; |
| C | at | rs6808574; |
| G | at | rs10010131; |
| A | at | rs10517086; |

(continued)

| | | |
|---|---|---|
| del G | at | rs371250843; |
| A | at | rs2524277; |
| G | at | rs9268645; |
| A | at | rs9269173; |
| T | at | rs17211699; |
| C | at | rs9273369; |
| A | at | rs10947332; |
| C | at | rs7454108; |
| dupTA | at | rs559242105; |
| C | at | rs9470794; |
| G | at | rs4407733; |
| A | at | rs4607517; |
| G | at | rs3996352; |
| A | at | rs3802177; |
| T | at | rs1574285; |
| G | at | rs7020673; |
| C | at | rs2104286; |
| C | at | rs4746890; |
| T | at | rs7903146; |
| T | at | rs2421016; |
| T | at | rs689; |
| T | at | rs7111341; |
| G | at | rs231362; |
| A | at | rs231361; |
| T | at | rs5215; |
| T | at | rs7944584; |
| C | at | rs1552224; |
| C | at | rs10219509; |
| A | at | rs7138803; |
| G | at | rs4759229; |
| T | at | rs653178; |
| A | at | rs7957197; |
| T | at | rs9585056; |
| T | at | rs2289702; |
| A | at | rs8042680; |
| G | at | rs12899811; |
| A | at | rs12444268; |
| A | at | rs9924471; |
| G | at | rs7202877; |
| G | at | rs45450798; |
| C | at | rs763361; |
| A | at | rs10423928; and |
| G | at | rs5763779, |

$p(Y_i=k|X_i)$ for k < K denotes the probability that the sample i has the outcome $k$ for k=1, $k$=2 and k=3, $p(Y_i=K|X_i)$ denotes the probability that the sample i has the outcome k=4.

In some embodiments, said input features further comprise the sex of the subject (e.g. male = 0, female = 1).

In some embodiments, the vector of regression coefficients $\beta_k$ for each outcome k corresponds to the set of regression coefficients for that outcome $k$ set forth in Table 3.

The methods of the invention can also be used to classify subjects into one of the three following groups: susceptibility to T1D, susceptibility to T2D and susceptibility to monogenic diabetes. In some cases, the patient may already have developed diabetes and the methods described herein are used to differentiate between T1D, T2D and monogenic diabetes, and therefore aid in providing an accurate diagnosis.

In some embodiments, the invention provides a method of classifying a subject into one of the groups selected from susceptibility to T1D, susceptibility to T2D and susceptibility to monogenic diabetes, the method comprising determining the identity of at least one allele of at least four (e.g. 4, 5, 6, 7 or 8) positions of single nucleotide polymorphism (SNP) selected from the following:

rs371250843;
rs9269173;
rs17211699;
rs2476601;
rs10947332;
rs7454108;
rs9924471; and
rs559242105,

and/or an SNP in linkage disequilibrium with any one of said SNPs at $r^2 > 0.8$, $r^2 > 0.9$, $r^2 > 0.95$, $r^2 > 0.99$ or $r^2 = 1.0$.

In some embodiments, the invention provides a method of classifying a subject into one of the groups selected from T1D, T2D and monogenic diabetes, the method comprising determining the identity of at least one allele at each of at least four positions (e.g. 4, 5, 6, 7 or 8) of single nucleotide polymorphism (SNP) selected from the following:

rs371250843;
rs9269173;
rs17211699;
rs2476601;
rs10947332;
rs7454108;
rs9924471; and
rs559242105,

and/or an SNP in linkage disequilibrium with any one of said SNPs at $r^2 > 0.8$, $r^2 > 0.9$, $r^2 > 0.95$, $r^2 > 0.99$ or $r^2 = 1.0$.

The methods may further comprise determining the identity of at least one allele of at least 1 SNP referred to in Table 4. For example, the method may further comprise determining the identity of 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55 or all of at least one allele of the SNPs referred to in Table 4.

The predictive allele associated with each of these SNPs is defined in Table 1. The presence of the predictive allele may determine the classification of the subject. The gene may be null, homozygous or heterozygous for the predictive allele.

In some embodiments, the methods may comprise determining the identity of at least one allele (e.g. one or two) from the following SNPs, wherein the presence of one or more of the following predictive alleles determines the classification of the subject:

| | | |
|---|---|---|
| G | at | rs2476601; |
| C | at | rs831571; |
| del G | at | rs371250843; |
| A | at | rs2524277; |
| A | at | rs9269173; |
| T | at | rs17211699; |
| A | at | rs10947332; |
| C | at | rs7454108; |
| dupTA | at | rs559242105; |
| C | at | rs9470794; |
| C | at | rs2104286; |
| T | at | rs7111341; |
| C | at | rs1552224; |
| C | at | rs10219509; |
| T | at | rs2289702; |

(continued)

| | | |
|---|---|---|
| A | at | rs9924471; |
| G | at | rs45450798; |
| C | at | rs10401969; |
| G | at | rs36608; |
| G | at | rs17791513; |
| T | at | rs16996148; |
| C | at | rs429358; |
| A | at | rs10503669; |
| G | at | rs1801282; |
| T | at | rs10203174; |
| T | at | rs9921586; |
| C | at | rs1531343; |
| G | at | rs10885122; |
| T | at | rs10923931; |
| C | at | rs7607980; |
| T | at | rs1106240; |
| T | at | rs17168486; |
| A | at | rs1031605; |
| G | at | rs35767; |
| T | at | rs10842994; |
| C | at | rs10811661; |
| C | at | rs17265513; |
| G | at | rs1182397; |
| T | at | rs10840595; |
| G | at | rs553668; |
| A | at | rs11920090; |
| A | at | rs4812829; |
| G | at | rs10146997; |
| T | at | rs61839660; |
| T | at | rs12189871; |
| A | at | rs144530872; |
| T | at | rs12251307; |
| T | at | rs16956936; |
| C | at | rs9259118; |
| T | at | rs72848653; |
| A | at | rs3024505; |
| C | at | rs425105; |
| T | at | rs2187668; |
| T | at | rs11258747; |
| A | at | rs2816316; |
| A | at | rs947474; |
| A | at | rs72928038; |
| C | at | rs17574546; |
| G | at | rs2327832; |
| T | at | rs3842753. |

In some embodiments, the method comprises determining the identity of the genotype of the subject at each SNP and inputting the genotype identities into a probability function to compute said classification.

The probability function may comprises a logistic regression algorithm. Each SNP may be associated with a specific weighting and the genotype data from a combination of SNPs can be used to classify subjects.

The logistic regression algorithm may also be based on other variables other than SNPs, such as sex of the subject.

In some embodiments, predicting diabetes status comprises carrying out a computer-implemented multinomial logistic regression to identify the diabetes status for the sample i having the highest probability from the group of diabetes statuses consisting of: susceptibility to T1D, susceptibility to T2D and susceptibility to monogenic diabetes, according to the following formulae (1) and (2):

$$p(Y_i = k | X_i) = \frac{e^{\beta_k \cdot X_i}}{1 + \sum_{j=1}^{K-1} e^{\beta_j \cdot X_i}}, k < K \qquad (1)$$

$$p(Y_i = K | X_i) = \frac{1}{1 + \sum_{j=1}^{K-1} e^{\beta_j \cdot X_i}} \qquad (2)$$

wherein:

K represents the three outcomes susceptibility to T1D (k=1), susceptibility to T2D (k=2), and susceptibility to monogenic diabetes (k=3; "the pivot outcome"),

$\beta_k$ denotes the vector of learned regression coefficients for the kth outcome, said regression coefficients comprising a coefficient for each input feature and an intercept value,

X denotes the vector of m input features of sample i, wherein said input features comprise the number of predictive alleles at each of at least the following SNPs:

| | | |
|---|---|---|
| G | at | rs2476601; |
| C | at | rs831571; |
| del G | at | rs371250843; |
| A | at | rs2524277; |
| A | at | rs9269173; |
| T | at | rs17211699; |
| A | at | rs10947332; |
| C | at | rs7454108; |
| dupTA | at | rs559242105; |
| C | at | rs9470794; |
| C | at | rs2104286; |
| T | at | rs7111341; |
| C | at | rs1552224; |
| C | at | rs10219509; |
| T | at | rs2289702; |
| A | at | rs9924471; |
| G | at | rs45450798; |
| C | at | rs10401969; |
| G | at | rs36608; |
| G | at | rs17791513; |
| T | at | rs16996148; |
| C | at | rs429358; |
| A | at | rs10503669; |
| G | at | rs1801282; |
| T | at | rs10203174; |
| T | at | rs9921586; |
| C | at | rs1531343; |
| G | at | rs10885122; |
| T | at | rs10923931; |
| C | at | rs7607980; |
| T | at | rs1106240; |
| T | at | rs17168486; |
| A | at | rs1031605; |
| G | at | rs35767; |

(continued)

| | | |
|---|---|---|
| T | at | rs10842994; |
| C | at | rs10811661; |
| C | at | rs17265513; |
| G | at | rs1182397; |
| T | at | rs10840595; |
| G | at | rs553668; |
| A | at | rs11920090; |
| A | at | rs4812829; |
| G | at | rs10146997; |
| T | at | rs61839660; |
| T | at | rs12189871; |
| A | at | rs144530872; |
| T | at | rs12251307; |
| T | at | rs16956936; |
| C | at | rs9259118; |
| T | at | rs72848653; |
| A | at | rs3024505; |
| C | at | rs425105; |
| T | at | rs2187668; |
| T | at | rs11258747; |
| A | at | rs2816316; |
| A | at | rs947474; |
| A | at | rs72928038; |
| C | at | rs17574546; |
| G | at | rs2327832; |
| T | at | rs3842753. |

$p(Y_i{=}k|X_i)$ for k < K denotes the probability that the sample i has the outcome $k$ for k=1 and k=2, $p(Y_i{=}K|X_i)$ denotes the probability that the sample i has the outcome k=3.

In some embodiments, predicting diabetes status comprises carrying out a computer-implemented multinomial logistic regression to identify the diabetes status for the sample i having the highest probability from the group of diabetes statuses consisting of: T1D, T2D and monogenic diabetes, according to the following formulae (1) and (2):

$$p(Y_i = k|X_i) = \frac{e^{\beta_k \cdot X_i}}{1+\sum_{j=1}^{K-1} e^{\beta_j \cdot X_i}}, k < K \qquad (1)$$

$$p(Y_i = K|X_i) = \frac{1}{1+\sum_{j=1}^{K-1} e^{\beta_j \cdot X_i}} \qquad (2)$$

wherein:
K represents the three outcomes T1D (k=1), T2D (k=2), and monogenic diabetes (k=3; "the pivot outcome"),
$\beta_k$ denotes the vector of learned regression coefficients for the kth outcome, said regression coefficients comprising a coefficient for each input feature and an intercept value,
$X_i$ denotes the vector of m input features of sample i, wherein said input features comprise the number of predictive alleles at each of at least the following SNPs:

| | | |
|---|---|---|
| G | at | rs2476601; |
| C | at | rs831571; |
| del G | at | rs371250843; |
| A | at | rs2524277; |
| A | at | rs9269173; |
| T | at | rs17211699; |

(continued)

| | | |
|---|---|---|
| A | at | rs10947332; |
| C | at | rs7454108; |
| dupTA | at | rs559242105; |
| C | at | rs9470794; |
| C | at | rs2104286; |
| T | at | rs7111341; |
| C | at | rs1552224; |
| C | at | rs10219509; |
| T | at | rs2289702; |
| A | at | rs9924471; |
| G | at | rs45450798; |
| C | at | rs10401969; |
| G | at | rs36608; |
| G | at | rs17791513; |
| T | at | rs16996148; |
| C | at | rs429358; |
| A | at | rs10503669; |
| G | at | rs1801282; |
| T | at | rs10203174; |
| T | at | rs9921586; |
| C | at | rs1531343; |
| G | at | rs10885122; |
| T | at | rs10923931; |
| C | at | rs7607980; |
| T | at | rs1106240; |
| T | at | rs17168486; |
| A | at | rs1031605; |
| G | at | rs35767; |
| T | at | rs10842994; |
| C | at | rs10811661; |
| C | at | rs17265513; |
| G | at | rs1182397; |
| T | at | rs10840595; |
| G | at | rs553668; |
| A | at | rs11920090; |
| A | at | rs4812829; |
| G | at | rs10146997; |
| T | at | rs61839660; |
| T | at | rs12189871; |
| A | at | rs144530872; |
| T | at | rs12251307; |
| T | at | rs16956936; |
| C | at | rs9259118; |
| T | at | rs72848653; |
| A | at | rs3024505; |
| C | at | rs425105; |
| T | at | rs2187668; |
| T | at | rs11258747; |
| A | at | rs2816316; |
| A | at | rs947474; |
| A | at | rs72928038; |
| C | at | rs17574546; |

(continued)

| | | |
|---|---|---|
| G | at | rs2327832; |
| T | at | rs3842753, |

$p(Y_i=k|X_i)$ for k < K denotes the probability that the sample i has the outcome k for k=1 and k=2, $p(Y_i=K|X_i)$ denotes the probability that the sample i has the outcome k=3.

In some embodiments, said input features further comprise the sex of the subject (e.g. male = 0, female = 1).

In some embodiments, the vector of regression coefficients $\beta_k$ for each outcome k corresponds to the set of regression coefficients for that outcome *k* set forth in Table 4.

Also provided is a system comprising:

a). a processor; and

b). a computer readable medium comprising instructions that, when executed by the processor, cause the processor to compute the classifications described above.

Also provided is a non-transitory computer readable medium or media comprising instructions that, when executed by at least one processor, cause the at least one processor to compute the classifications described above.

*Prediction of T1D susceptibility*

The methods of the invention can also be used to screen for likelihood of a subject developing T1D. Screening for predisposition to T1D allows individuals who are identified as high risk to prepare and even delay or prevent onset of the disease. For example, patients who are identified as high risk may be monitored and administered treatments to delay onset, such as teplizumab. The present methods allow screening even before autoantibodies can be detected and the methods which are efficient, cost-effective and non-invasive are applicable to individuals of all ages.

In some embodiments, predicting diabetes status comprises assessing the probability that a subject will develop T1D compared to T2D, monogenic diabetes and/or no diabetes.

Thus, the invention provides a method of assessing the probability that a subject will develop T1D compared to T2D, monogenic diabetes and/or no diabetes, the method comprising determining the identity of at least one allele at each of at least four (e.g. 4, 5, 6, 7 or 8) positions of single nucleotide polymorphism (SNP) selected from the following:

rs371250843;
rs9269173;
rs17211699;
rs2476601;
rs10947332;
rs7454108;
rs9924471; and
rs559242105,

and/or an SNP in linkage disequilibrium with any one of said SNPs at $r^2 > 0.8$, $r^2 > 0.9$, $r^2 > 0.95$, $r^2 > 0.99$ or $r^2 = 1.0$.

The method may further comprise determining the identity of at least one allele of at least 1 SNP referred to in Table 8. For example, the method may further comprise determining the identity of 2, 3, 4, 5, 6, 7, 8, 9, 10, 11, 12 or 13 of at least one allele of the SNPs referred to in Table 8.

The predictive allele associated with each of these SNPs is defined in Table 1. The presence and occurrence of the predictive allele may determine the risk of the subject developing T1D. The gene may be null, homozygous or heterozygous for the predictive allele.

In some embodiments, predicting diabetes status comprises assessing the probability that a subject will develop T1D compared to T2D, monogenic diabetes and no diabetes. In other words, assessing the probability that a subject will develop T1D compared to not developing T1D.

Thus, the invention also provides a method for assessing the probability that a subject will develop T1D compared to T2D, monogenic diabetes and no diabetes, the method comprising determining the identity of at least one allele at each of at least four (e.g. 4, 5, 6, 7 or 8) positions of single nucleotide polymorphism (SNP) selected from the following:

rs371250843;
rs9269173;

rs17211699;
rs2476601;
rs10947332;
rs7454108;
rs9924471; and
rs559242105,

and/or an SNP in linkage disequilibrium with any one of said SNPs at $r^2 > 0.8$, $r^2 > 0.9$, $r^2 > 0.95$, $r^2 > 0.99$ or $r^2 = 1.0$.

The method may further comprise determining the identity of at least one allele of at least 1 SNP referred to in Table 8. For example, the method may further comprise determining the identity of 2, 3, 4, 5, 6, 7, 8, 9, 10, 11, 12 or 13 of at least one allele of the SNPs referred to in Table 8.

The predictive allele associated with each of these SNPs is defined in Table 1. The presence and occurrence of the predictive allele may determine risk of the subject developing T1D. The gene may be null, homozygous or heterozygous for the predictive allele.

In some embodiments, the methods may comprise determining the identity of at least one allele (e.g. one or two) from the following SNPs, wherein the presence of one or more of the following predictive alleles determines the risk of a subject developing T1D compared to T2D, monogenic diabetes and no diabetes:

| | | |
|---|---|---|
| G | at | rs2476601 |
| del G | at | rs371250843 |
| A | at | rs2524277 |
| G | at | rs9268645 |
| A | at | rs9269173 |
| T | at | rs17211699 |
| A | at | rs10947332 |
| C | at | rs7454108 |
| dupTA | at | rs559242105 |
| A | at | rs9924471 |
| T | at | rs2187668 |
| T | at | rs3842753 |
| T | at | rs4788084. |

In some embodiments, predicting diabetes status comprises assessing the probability that a subject will develop T1D compared to T2D.

The invention also provides a method for assessing the probability that a subject will develop T1D compared to T2D, the method comprising determining the identity of at least one allele at each of at least four (e.g. 4, 5, 6, 7 or 8) positions of single nucleotide polymorphism (SNP) selected from the following:

rs371250843;
rs9269173;
rs17211699;
rs2476601;
rs10947332;
rs7454108;
rs9924471; and
rs559242105,

and/or an SNP in linkage disequilibrium with any one of said SNPs at $r^2 > 0.8$, $r^2 > 0.9$, $r^2 > 0.95$, $r^2 > 0.99$ or $r^2 = 1.0$.

In some embodiments, predicting diabetes status comprises assessing the probability that a subject will develop T1D compared to monogenic diabetes.

Thus, the invention also provides a method for assessing the probability that a subject will develop T1D compared to monogenic diabetes, the method comprising determining the identity of at least one allele at each of at least four (e.g. 4, 5, 6, 7 or 8) positions of single nucleotide polymorphism (SNP) selected from the following:

rs371250843;
rs9269173;

rs17211699;
rs2476601;
rs10947332;
rs7454108;
rs9924471; and
rs559242105,

and/or an SNP in linkage disequilibrium with any one of said SNPs at $r^2 > 0.8$, $r^2 > 0.9$, $r^2 > 0.95$, $r^2 > 0.99$ or $r^2 = 1.0$.

The method may further comprise determining the identity of at least one allele of at least 1 SNP referred to in Table 10. For example, the method may further comprise determining the identity of 2, 3, 4, 5, 6, 7, 8, 9, 10, 11, 12, 13, 14, 15, 16, 17, or 18 of at least one allele of the SNPs referred to in Table 10.

The predictive allele associated with each these SNPs is defined in Table 1. The presence and occurrence of the predictive allele may determine risk of the subject developing T1D. The gene may be null, homozygous or heterozygous for the predictive allele.

In some embodiments, the methods may comprise determining the identity of at least one allele (e.g. one or two) from the following SNPs, wherein the presence of one or more of the following predictive alleles determines the risk of a subject developing T1D compared to monogenic diabetes:

| | | |
|---|---|---|
| G | at | rs2476601 |
| C | at | rs831571 |
| del G | at | rs371250843 |
| G | at | rs9268645 |
| A | at | rs9269173 |
| T | at | rs17211699 |
| C | at | rs9273369 |
| A | at | rs10947332 |
| C | at | rs7454108 |
| dupTA | at | rs559242105 |
| A | at | rs4607517 |
| T | at | rs7111341 |
| G | at | rs4759229 |
| T | at | rs2289702 |
| A | at | rs9924471 |
| G | at | rs5763779 |
| T | at | rs3842753 |
| C | at | rs3184504. |

In some embodiments of the methods described above, the method comprises determining the identity of the genotype of the subject at each SNP, and further comprises computing a T1D risk score for said subject based on the identity of the genotype at each SNP.

In some embodiments, the method comprises inputting the genotype identities into a probability function to compute said risk score. The probability function may comprise a logistic regression algorithm. Each SNP may be associated with a specific weighting and the genotype data from a combination of SNPs can be used to determine the risk score. The logistic regression algorithm may also be based on other variables other than SNPs, such as sex of the subject.

In some embodiments, computing the risk score from the genotype data comprises weighting the contribution of each SNP found to be present such that the contribution of each SNP to the risk score is commensurate with an odds ratio for the association of the SNP to type 1 diabetes, as set forth in Tables 8 or 10.

In some embodiments, computing said risk score comprises using the following formula:

$$risk\ score\ \ p = \frac{1}{1 + e^{-(-\beta_0 + \sum_{i=1}^{m} \beta_i x_i)}}$$

wherein $\beta 0$ is an intercept weight, $\beta$ is a vector of weights for each of m variables and x is a vector of variables associated with the subject, wherein the variables comprise the number of predictive alleles present at each SNP and optionally variables derived therefrom.

When predicting diabetes status comprises assessing T1D compared to T2D, monogenic diabetes and no diabetes, computing said risk score may comprise using the following formula:

$$risk\ score\ \ p = \frac{1}{1 + e^{-(-\beta_0 + \sum_{i=1}^{m} \beta_i x_i)}}$$

wherein $\beta0$ is an intercept weight, $\beta$ is a vector of weights for each of m variables and x is a vector of variables associated with the subject, wherein the variables comprise the number of predictive alleles present at each of the following SNPs:

| | | |
|---|---|---|
| G | at | rs2476601 |
| del G | at | rs371250843 |
| A | at | rs2524277 |
| G | at | rs9268645 |
| A | at | rs9269173 |
| T | at | rs17211699 |
| A | at | rs10947332 |
| C | at | rs7454108 |
| dupTA | at | rs559242105 |
| A | at | rs9924471 |
| T | at | rs2187668 |
| T | at | rs3842753 |
| T | at | rs4788084, |

and optionally variables derived therefrom.

When predicting diabetes status comprises assessing T1D compared to monogenic diabetes, computing said risk score may comprise using the following formula:

$$risk\ score\ \ p = \frac{1}{1 + e^{-(-\beta_0 + \sum_{i=1}^{m} \beta_i x_i)}}$$

wherein $\beta0$ is an intercept weight, $\beta$ is a vector of weights for each of m variables and x is a vector of variables associated with the subject, wherein the variables comprise the number of predictive alleles present at each of the following SNPs:

| | | |
|---|---|---|
| G | at | rs2476601 |
| C | at | rs831571 |
| del G | at | rs371250843 |
| G | at | rs9268645 |
| A | at | rs9269173 |
| T | at | rs17211699 |
| C | at | rs9273369 |
| A | at | rs10947332 |
| C | at | rs7454108 |
| dupTA | at | rs559242105 |
| A | at | rs4607517 |
| T | at | rs7111341 |
| G | at | rs4759229 |
| T | at | rs2289702 |
| A | at | rs9924471 |
| G | at | rs5763779 |
| T | at | rs3842753 |
| C | at | rs3184504, |

and optionally variables derived therefrom.

Also provided is a system comprising:

a). a processor; and

b). a computer readable medium comprising instructions that, when executed by the processor, cause the processor to compute a T1D risk score as described above.

Also provided is a non-transitory computer readable medium or media comprising instructions that, when executed by at least one processor, cause the at least one processor to computing a T1D risk score as described above.

### *Treatment* **of** *diabetes*

The methods described above provide the classification or risk of a subject developing a specific type of diabetes. Once a subject has been determined to be at risk of diabetes or is determined to have diabetes, further tests can be carried out and treatments given based on the type of diabetes.

In the methods, following identification of the at least one allele at each single nucleotide polymorphism (SNP), if the subject is determined to be at risk of developing Type 1 Diabetes, the method may further comprise administering to the subject a test to screen for type 1 diabetes. Such tests are known to the skilled person. For example, the test may be selected from the group consisting of: detection of type 1 diabetes autoantibodies, a glycated hemoglobin (A1C) test, a blood sugar test or a C-peptide test.

Where the test is detection of type 1 diabetes autoantibodies, any autoantibody associated type 1 diabetes can be detected. Examples include detection of islet cell autoantibodies (ICA), glutamic acid decarboxylase autoantibodies (GAD-65), insulin autoantibodies (IAA) and protein tyrosine phosphatase autoantibodies (IA-2A). The test could be administered once or more than once following the determination that the subject is at risk of type 1 diabetes. For example, the test could be administered at several time points after determination that the subject is at risk of type 1 diabetes.

Where the test is a blood sugar test, it may be a fasting blood sugar test or a blood sugar test taken at a random time regardless of food intake.

T1D is caused by the destruction of β-cells such that insulin is no longer produced by the body. Individuals with the disease therefore have to continually monitor their blood sugar levels and require continual injection of insulin. Severe symptoms of T1D include hypoglycaemia and ketoacidosis, which can be life-threatening. T1D is a chronic condition, of which there is currently no cure. Therefore, delaying onset of the disease is invaluable to patients. By assessing susceptibility to T1D early, this allows for preparation and opens the window for the use of drugs which can prevent or delay the onset of disease.

Accordingly, in some embodiments, the disclosure provides a method of preventing or delaying onset of Type 1 Diabetes in a subject, the method comprising determining the identity of at least one allele at each of at least four (e.g. 4, 5, 6, 7 or 8) positions of single nucleotide polymorphism (SNP) selected from the following:

rs371250843;
rs9269173;
rs17211699;
rs2476601;
rs10947332;
rs7454108;
rs9924471; and
rs559242105,

and/or an SNP in linkage disequilibrium with any one of said SNPs at $r^2 > 0.8$, $r^2 > 0.9$, $r^2 > 0.95$, $r^2 > 0.99$ or $r^2 = 1.0$; determining the subject to be at risk of developing T1D, and administering a medicament for preventing or delaying the onset of T1D.

The method may further comprise determining the identity of at least one allele of at least one (e.g. 1, 2, 3, 4 or 5) of the SNPs selected from:

rs2524277;

rs9268645;

rs2187668;

rs3842753; and

rs4788084,

and/or an SNP in linkage disequilibrium with any one of said SNPs at $r^2 > 0.8$, $r^2 > 0.9$, $r^2 > 0.95$, $r^2 > 0.99$ or $r^2 = 1.0$.

In some embodiments, provided is a method for preventing or delaying onset of Type 1 Diabetes in a subject, the method comprising determining the identity of at least one allele (e.g. one or two) from the following SNPs, wherein the presence of one or more of the following predictive alleles determines the risk of a subject developing T1D:

| | | |
|---|---|---|
| G | at | rs2476601 |
| del G | at | rs371250843 |
| A | at | rs2524277 |
| G | at | rs9268645 |
| A | at | rs9269173 |
| T | at | rs17211699 |
| A | at | rs10947332 |
| C | at | rs7454108 |
| dupTA | at | rs559242105 |
| A | at | rs9924471 |
| T | at | rs2187668 |
| T | at | rs3842753 |
| T | at | rs4788084; |

determining the subject to be at risk of developing T1D, and administering a medicament for preventing or delaying the onset of T1D.

In some embodiments, provided is a method for preventing or delaying onset of Type 1 Diabetes in a subject, the method comprising determining the identity of at least one allele (e.g. one or two) from the following SNPs, wherein the presence of one or more of the following predictive alleles determines the risk of a subject developing T1D:

| | | |
|---|---|---|
| G | at | rs2476601 |
| C | at | rs831571 |
| del G | at | rs371250843 |
| G | at | rs9268645 |
| A | at | rs9269173 |
| T | at | rs17211699 |
| C | at | rs9273369 |
| A | at | rs10947332 |
| C | at | rs7454108 |
| dupTA | at | rs559242105 |
| A | at | rs4607517 |
| T | at | rs7111341 |
| G | at | rs4759229 |
| T | at | rs2289702 |
| A | at | rs9924471 |
| G | at | rs5763779 |
| T | at | rs3842753 |
| C | at | rs3184504, |

determining the subject to be at risk of developing T1D, and administering a medicament for preventing or delaying the onset of T1D.

In some embodiments, the medicament for preventing or delaying the onset of T1D is Teplizumab.

If the subject is determined to be susceptible to or have T2D, they can be treated by methods known in the art. Examples of treatments and advice include insulin and metformin, eating well and exercise.

Accordingly, in some embodiments, the disclosure provides a method of treating Type 2 Diabetes in a subject, the method comprising determining the identity of at least one allele at each of at least four (e.g. 4, 5, 6, 7 or 8) positions of single nucleotide polymorphism (SNP) selected from the following:

rs371250843;
rs9269173;
rs17211699;
rs2476601;
rs10947332;
rs7454108;
rs9924471; and
rs559242105,

and/or an SNP in linkage disequilibrium with any one of said SNPs at $r^2 > 0.8$, $r^2 > 0.9$, $r^2 > 0.95$, $r^2 > 0.99$ or $r^2 = 1.0$; determining the subject to have T2D, and administering a medicament for treating T2D. The medicament may be insulin and/or metformin.

If the subject is determined to be susceptible to or have monogenic diabetes, they can be treated by methods known in the art. Examples of treatments insulin and sulfonylurea.

Accordingly, in some embodiments, the disclosure provides a method of treating monogenic diabetes in a subject, the method comprising determining the identity of at least one allele at each of at least four (e.g. 4, 5, 6, 7 or 8) positions of single nucleotide polymorphism (SNP) selected from the following:

rs371250843;
rs9269173;
rs17211699;
rs2476601;
rs10947332;
rs7454108;
rs9924471; and
rs559242105,

and/or an SNP in linkage disequilibrium with any one of said SNPs at $r^2 > 0.8$, $r^2 > 0.9$, $r^2 > 0.95$, $r^2 > 0.99$ or $r^2 = 1.0$; determining the subject to have monogenic diabetes, and administering a medicament for treating monogenic diabetes. The medicament may be insulin and/or sulfonylurea.

***Genotyping Assays***

Aspects of the invention relate to determining the presence of SNPs through obtaining a patient DNA sample and evaluating the patient sample for the presence of three or more SNPs. It should be appreciated that a patient DNA sample can be extracted, and a SNP can be detected in the sample, through any means known to one of ordinary skill in art. Some non-limiting examples of known techniques include detection via restriction fragment length polymorphism (RFLP) analysis, planar microarrays, bead arrays, sequencing, single strand conformation polymorphism analysis (SSCP), chemical cleavage of mismatch (CCM), and denaturing high performance liquid chromatography (DHPLC).

In some embodiments, a SNP is detected through PCR amplification and sequencing of the DNA region comprising the SNP. In some embodiments SNPs are detected using microarrays. Microarrays for detection of genetic polymorphisms, changes or mutations (in general, genetic variations) such as a SNP in a DNA sequence, comprise a solid surface, typically glass, on which a high number of genetic sequences are deposited (the probes), complementary to the genetic variations to be studied. Using standard robotic printers to apply probes to the array a high density of individual probe features can be obtained, for example probe densities of 600 features per cm2 or more can be typically achieved. The positioning of probes on an array is precisely controlled by the printing device (robot, inkjet printer, photolithographic mask etc) and probes are aligned in a grid. The organisation of probes on the array facilitates the subsequent identification of specific probe-target interactions. Additionally it is common, but not necessary, to divide the array features into smaller sectors, also grid-shaped, that are subsequently referred to as sub-arrays. Sub-arrays typically comprise 32 individual probe features although lower (e.g. 16) or higher (e.g. 64 or more) features can comprise each subarray.

In some embodiments, detection of genetic variation such as the presence of a SNP involves hybridization to sequences which specifically recognize the normal and the predictive allele in a fragment of DNA derived from a test sample. Typically, the fragment has been amplified, e.g. by using the polymerase chain reaction (PCR), and labelled e.g. with a fluorescent molecule. A laser can be used to detect bound labelled fragments on the chip and thus an individual who is homozygous for the normal allele can be specifically distinguished from heterozygous individuals (in the case of autosomal dominant conditions then these individuals are referred to as carriers) or those who are homozygous for the risk allele. In some embodiments, the amplification reaction and/or extension reaction is carried out on the microarray or bead itself.

In some embodiments, methods described herein may involve hybridization. For differential hybridization based methods there are a number of methods for analysing hybridization data for genotyping:

Increase in hybridization level: The hybridization levels of probes complementary to the normal and mutant alleles are compared.

Decrease in hybridization level: Differences in the sequence between a control sample and a test sample can be identified by a decrease in the hybridization level of the totally complementary oligonucleotides with a reference sequence. A loss approximating 100% is produced in mutant homozygous individuals while there is only an approximately 50% loss in heterozygotes. In Microarrays for examining all the bases of a sequence of "n" nucleotides ("oligonucleotide") of length in both strands, a minimum of "2n" oligonucleotides that overlap with the previous oligonucleotide in all the sequence except in the nucleotide are necessary. Typically the size of the oligonucleotides is about 25 nucleotides. However it should be appreciated that the oligonucleotide can be any length that is appropriate as would be understood by one of ordinary skill in the art. In particular, the use of a minor groove binding domain (MBD) permits shorter probe sequences while retaining high discrimination between the perfect match and the mismatch. The increased number of oligonucleotides used to reconstruct the sequence reduces errors derived from fluctuation of the hybridization level. However, the exact change in sequence cannot be identified with this method; in some embodiments this method is combined with sequencing to identify the mutation.

Where amplification or extension is carried out on the microarray or bead itself, three methods are presented by way of example:

In the Minisequencing strategy, a mutation specific primer is fixed on the slide and after an extension reaction with fluorescent dideoxynucleotides, the image of the Microarray is captured with a scanner.

In the Primer extension strategy, two oligonucleotides are designed for detection of the wild type and mutant sequences respectively. The extension reaction is subsequently carried out with one fluorescently labelled nucleotide and the remaining nucleotides unlabelled. In either case the starting material can be either an RNA sample or a DNA product amplified by PCR.

In the Tag arrays strategy, an extension reaction is carried out in solution with specific primers, which carry a determined 5' sequence or "tag". The use of Microarrays with oligonucleotides complementary to these sequences or "tags" allows the capture of the resultant products of the extension. Examples of this include the high density Microarray "Flex-flex" (Affymetrix).

For cost-effective genetic diagnosis, in some embodiments, the need for amplification and purification reactions presents disadvantages for the on-chip or on-bead extension/amplification methods compared to the differential hybridization based methods. However the techniques may still be used to detect and diagnose conditions according to the invention.

Typically, Microarray or bead analysis is carried out using differential hybridization techniques. However, differential hybridization does not produce as high specificity or sensitivity as methods associated with amplification on glass slides. For this reason the development of mathematical algorithms, which increase specificity and sensitivity of the hybridization methodology, are needed (Cutler DJ, Zwick ME, Carrasquillo MN, Yohn CT, Tobi KP, Kashuk C, Mathews DJ, Shah N, Eichler EE, Warrington JA, Chakravarti A. Genome Research; 11 :1913-1925 (2001). Methods of genotyping using microarrays and beads are known in the art.

The genotyping platform for use in the methods of the present invention may be based on the TaqMan® OpenArray® SNP Genotyping system available from Life Technologies. Further details of the TaqMan® genotyping system and OpenArray® format are available from the Life Technologies, Applied Biosystems, webpage, e.g., the TaqMan® OpenArray® Genotyping Getting Started Guide, © 2010 Life Technologies Corporation.

Alternatively or additionally, the genotyping platform for use in the methods of the present invention may be based on the Dynamic Array IFCs Genotyping System from Fluidigm. Further details of the Dynamic Array IFCs Genotyping System are available from Fluidigm webpage.

In a fifth aspect, the invention provides a genotyping tool for use in the methods described herein, said tool comprising an array having a plurality of oligonucleotide probe pairs, each of said probe pairs comprising a first probe specific for a first allele of a single nucleotide polymorphism (SNP) and a second probe specific for a second allele of the SNP, wherein said plurality of oligonucleotide probe pairs comprises probe pairs that interrogate at least four (e.g. 4, 5, 6, 7 or 8) of the following SNPs:

rs371250843;

rs9269173;

rs17211699;

rs2476601;

rs10947332;

rs7454108;

rs9924471; and

rs559242105,

and/or an SNP in linkage disequilibrium with any one of said SNPs at $r^2 > 0.8$, $r^2 > 0.9$, $r^2 > 0.95$, $r^2 > 0.99$ or $r^2 = 1.0$.

The genotyping tool may further comprising oligonucleotide probe pairs that interrogate at least one (e.g. 1, 2, 3, 4 or 5) of the SNPs selected from:

rs2524277;
rs9268645;
rs2187668;
rs3842753; and
rs4788084,

and/or an SNP in linkage disequilibrium with any one of said SNPs at $r^2 > 0.8$, $r^2 > 0.9$, $r^2 > 0.95$, $r^2 > 0.99$ or $r^2 = 1.0$.

For example, the genotyping tool may comprise oligonucleotide probe pairs that interrogate rs371250843, rs9269173, rs17211699, rs2187668 and rs559242105.

In another example, the genotyping tool may comprise oligonucleotide probe pairs that interrogate rs3842753, rs2281389, rs9268645, rs2476601, rs2524277, rs9924471, rs7454108, rs10947332 and rs4788084. Note that rs2281389 is in linkage disequilibrium with rs559242105.

In another example, the genotyping tool may comprise oligonucleotide probe pairs that interrogate rs2476601, rs371250843, rs2524277, rs9268645, rs9269173, rs17211699, rs10947332, rs7454108, rs559242105, rs9924471, rs2187668, rs3842753 and rs4788084.

In some embodiments, the oligonucleotide probes of the array that interrogate SNPs selected from rs371250843, rs9269173, rs17211699, rs2476601, rs10947332, rs7454108, rs9924471 and rs559242105 make up at least 50%, at least 60%, at least 70%, at least 80%, at least 90%, at least 95%, at least 99% of the total number of nucleic acid probes in the array, or essentially all of the nucleic acid probes in the array. In this way the genotyping tool is enriched for probes that interrogate SNPs informative for diabetes status (e.g. type 1 diabetes risk prediction). By avoiding a high proportion of probes that interrogate other SNPs (e.g. as is typically seen in large-scale SNP microarrays), the genotyping tool of the present invention may provide a more efficient tool for classification of diabetes type or assessment of type 1 diabetes risk prediction whereby use of unnecessary probes and other reagents is minimized.

In the genotyping tool, the total number of different SNPs for which allele-specific probes are provided may not exceed 100, 90, 80, 70, 60, 50, 40, 30, 25, 20, 19, 18, 17, 16, 15, 14, or 13.

The genotyping tool may be in the form of a TaqMan® OpenArray® SNP genotyping platform, a Dynamic Array integrated fluidic circuits (IFC) genotyping platform, a next-generation sequencing system, or a MassARRAY® system.

In the genotyping tool, the allele-specific oligonucleotide probes may each be covalently attached to a fluorophore, a quencher and/or to a minor groove binding domain (MGB). Each member of an allele-specific probe pair may be conjugated to a different fluorophore enabling specific detection of the probe pair members by fluorescence wavelength.

In some cases, the tool may further comprise a primer pair for each of said SNPs, said primer pair for each SNP comprising an oligonucleotide primer that hybridizes to a target sequence upstream of the SNP and an oligonucleotide primer that hybridizes to a target sequence downstream of the SNP.

In some cases, the tool may further comprise one or more reagents for amplification of DNA comprising said SNPs and/or for detection of said allele-specific probes.

***Examples***

*EXAMPLE 1*

Recent studies affirm that genetics can have a great influence on the appearance of different types of diabetes [3], [4], [5]. These studies are based on new methods of massive genetic analysis that analyze multiple Single Nucleotide Polymorphisms (SNPs), that is, variations in a single nucleotide to which a disease can be associated. Furthermore, since the latest technological advances in this area allow the rapid sequencing and obtaining of these variants and the subsequent analysis of this data, it is possible to create Polygenic Risk Scores (PRS). These are tools capable of predicting the probability that a patient has of suffering from a type of diabetes through Machine Learning (ML) algorithms that process the aforementioned polymorphic variants.

Accordingly, a machine learning algorithm was developed to differentiate the probability of an individual developing T1D, T2D, MODY or no diabetes.

## 1.1 Cohorts studied

Data comprising all of the SNPs of different patients, along with various annotations was obtained from Biocruces. The samples were from T1D patients, T2D patients, MODY patients and controls. The T1D and MODY samples from this cohort belong to young patients and were recruited by Biocruces in the genetics and diabetes laboratory directed by Dr. Luis Castaño. T2D patients and controls, on the other hand, were adults who had participated in the Di@bet.es study. The controls were over 70 years old, to have greater certainty that they will not develop T2D with age. The number of patients in each group is outlined in Table 2 below. 70% of Biocruces data was used to train the subsequent machine learning model and 30% was used to validate it.

Table 2: Cohorts studied

| Diagnosis | No. of patients |
|-----------|-----------------|
| T1D | 486 |
| MODY | 486 |
| T2D | 464 |
| Control | 475 |

## 1.2 Data processing and attribute selection

The data obtained from Biocruces comprised approximately 14 million SNPs and therefore a smaller selection of 305 SNPs was made. These 305 SNPs included SNPs associated with T1D and T2D. The controls and T2D groups also shared 154 clinical attributes. The only clinical attribute present in all four groups was "sex".

Two models were created. The first included data from T1D, T2D, MODY and control patients. The second included data from only T1D, T2D and MODY patients. However the methodology followed to create both models was the same.

### 1.21 Data preprocessing

When preparing the data for the subsequent steps, it was first ensured that the classes were balanced and that there were no missing values. Furthermore, all attributes were categorical, so it was not necessary to normalize any. Since the attribute "sex" was common to all four groups, this was used in the machine learning models. The "sex" attribute was given the values 0 (men) and 1 (women), while the SNPs could have a value of 0, 1 or 2, depending on the number of different alleles with respect to the reference genome.

### 1.22 Attribute selection

Once the data was ready for processing, the most significant attributes for the model to be developed were selected. To begin with, attributes with a frequency of <10% or >90% in the cohort were filtered so that the model could be more easily transferred to the general population.

The attribute selection algorithms were then applied. In this case, since the idea was to classify 3 or 4 groups and all the attributes were categorical, only the Chi square test was used, and it was not necessary to correct the p values, since none of the frequencies of the values of each group for the different attributes was less than 5. The working of this algorithm is explained in detail below.

The Chi square test is very similar to Fisher's exact test and is carried out when it is desired that the dimension of the contingency tables be greater than 2x2. In this case, it has been used to evaluate the association of the different categorical attributes of the "Diabet2prevent" algorithm that has been developed to compare the four groups at the same time.

Regarding the operation of this test, initially, the initial hypothesis is raised that the attributes are independent with respect to the groups that are to be predicted. The p-value of each attribute is then calculated. To do this, a contingency table is created for each attribute, and taking into account the marginal values, the theoretical frequencies of each box are calculated and compared with those observed in said table. Taking this into account, the p value indicates the probability of obtaining a difference equal to or greater than that observed in each table assuming that the initial hypothesis is true. Therefore, if a very low p value is obtained, the initial hypothesis is rejected [26].

Then, in order to avoid linkage disequilibrium and eliminate attributes that provide redundant information, the Pearson correlation test was carried out.

Before continuing with the next phase, it is important to clarify that by carrying out both the Chi square test and the Pearson correlation of the attributes of the PRS for T1D, T2D and monogenic diabetes, a total of 201 attributes were obtained. This number being too high, the 60 most significant of these were selected, taking into account the type of diabetes to which these SNPs were associated. Therefore, 30 SNPs associated with T1D and T2D were selected. The database did not include any SNP that is known to be associated with monogenic diabetes. This entire process was developed with the ScyPy and Sklearn libraries, and the final selection is shown in Tables 3 and 4.

*1.3 Selection of machine learning algorithm and its hyperparameters*

After selecting the most significant attributes for the two models, the selection of the ML algorithm was carried out. It was of interest to obtain the probability that a patient belongs to the different groups (T1D, T2D, monogenic or control). To know the weight that each attribute has in the result and create a model that is easy to transfer to future databases, it was decided to use the Logistic Regression algorithm for both models. Since more than two groups are being compared, the Logistic Regression is multinomial.

Multinomial logistic regression returns the probability that a patient has of belonging to each of the classes, so per sample as many probabilities as there are classes are obtained. These are subsequently classified depending on which is the largest.

These probabilities have to maintain a relationship between them, since otherwise it is possible that the model would give a high probability for more than one group, or for none. Therefore, the sum of the probabilities of all groups for each sample will always be 1. To compare four classes, the probabilities are calculated as follows [44]:

$$p(group\ 0) = ey0\ ey0 + ey1 + ey2 + ey3$$

$$p(group\ 1) = ey1\ ey0 + ey1 + ey2 + ey3$$

$$p(group\ 2) = ey2\ ey0 + ey1 + ey2 + ey3$$

$$p(group\ 3) = ey3\ ey0 + ey1 + ey2 + ey3$$

It is important to mention that, as in Logistic Regression, the y refers to a linear equation that relates the attributes to the group to be predicted.

To finish defining the algorithm of the models, the most appropriate hyperparameters were established for each of them. Multinomial Logistic Regression has the same hyperparameters as Binomial Logistic Regression (Logistic Regression Hyperparameters), and the value of each of them for each model is shown in Table 5. The coefficients that the models have assigned for each attribute are shown in Tables 3 and 4. These models have also been carried out with the Sklearn library.

Table 3: Regression coefficients of selected attributes for control, T1 D, T2D and monogenic diabetes.

| Attribute | P value | Logistic regression coefficient | | | |
|---|---|---|---|---|---|
| | | **CONTROL (0)** | **T1D (1)** | **T2D (2)** | **MONOGENIC diabetes(3)** |
| | | Intercept $\beta_0$ = 0,2999 | Intercept $\beta_0$ = -1,1427 | Intercept $\beta_0$ = 0,3259 | Intercept $\beta_0$ = 0,6069 |
| **SEX** | 3,77E-05 | 0,1747 | -0,0631 | -0,1649 | 0,0533 |
| **rs2476601** | 1,65E-06 | -0,0206 | 0,1570 | -0,1156 | -0,0208 |

(continued)

| Attribute | P value | Logistic regression coefficient | | | |
|---|---|---|---|---|---|
| | | CONTROL (0) | T1D (1) | T2D (2) | MONOGENIC diabetes(3) |
| | | Intercept $\beta_0$ = 0,2999 | Intercept $\beta_0$ = -1,1427 | Intercept $\beta_0$ = 0,3259 | Intercept $\beta_0$ = 0,6069 |
| rs782810 | 0,0309 | -0,0936 | 0,0780 | -0,0398 | 0,0554 |
| rs6742799 | 0,0167 | 0,0026 | 0,0199 | -0,1269 | 0,1045 |
| rs560887 | 0,0335 | 0,0337 | 0,0509 | 0,0401 | -0,1247 |
| rs1496653 | 0,0113 | 0,1043 | 0,0547 | -0,1811 | 0,0221 |
| rs831571 | 0,0207 | 0,0389 | 0,1866 | -0,0339 | -0,1917 |
| rs4402960 | 0,0061 | -0,0581 | -0,0468 | 0,0225 | 0,0824 |
| rs7649121 | 0,0265 | -0,0112 | 0,1478 | -0,0520 | -0,0846 |
| rs6808574 | 0,0098 | 0,0588 | 0,0504 | 0,0318 | -0,1411 |
| rs10010131 | 0,0489 | 0,1277 | -0,0621 | -0,0848 | 0,0191 |
| rs10517086 | 0,0432 | -0,0101 | 0,0824 | -0,0178 | -0,0544 |
| rs371250843 | 5,42E-17 | -0,0248 | 0,0993 | -0,1781 | 0,1037 |
| rs2524277 | 0,0076 | -0,0077 | -0,0162 | 0,0264 | -0,0024 |
| rs9268645 | 3,75E-19 | -0,0741 | 0,0498 | 0,0174 | 0,0069 |
| rs9269173 | 1,65E-47 | -0,0580 | 0,2743 | -0,1464 | -0,0699 |
| rs17211699 | 5,91E-08 | -0,0183 | -0,0678 | 0,1101 | -0,0240 |
| rs9273369 | 3,44E-67 | -0,2756 | 0,6642 | -0,1654 | -0,2232 |
| rs10947332 | 1,54E-04 | -0,0188 | -0,0467 | -0,0116 | 0,0772 |
| rs7454108 | 1,32E-65 | -0,3301 | 0,8771 | -0,2255 | -0,3215 |
| rs559242105 | 1,65E-05 | -0,0513 | 0,1099 | -0,1377 | 0,0791 |
| rs9470794 | 0,0340 | 0,1042 | 5,61E-04 | -0,0986 | -0,0062 |
| rs4407733 | 0,0486 | 0,0320 | -0,0628 | -0,0294 | 0,0603 |
| rs4607517 | 0,0013 | 0,0509 | -0,1791 | 0,0252 | 0,1030 |
| rs3996352 | 0,0082 | 0,0075 | -0,0213 | -0,0258 | 0,0396 |
| rs3802177 | 0,0055 | 0,0212 | 0,1630 | -0,2397 | 0,0555 |
| rs1574285 | 0,0381 | -0,0853 | 0,0295 | -0,0376 | 0,0935 |
| rs7020673 | 0,0173 | 0,0599 | -0,0557 | 0,0863 | -0,0906 |
| rs2104286 | 0,0409 | 0,0905 | 0,0308 | -0,0867 | -0,0345 |
| rs4746890 | 0,0497 | 0,0379 | 0,0299 | -0,0750 | 0,0073 |
| rs7903146 | 0,0180 | -0,0189 | -0,1085 | 0,2356 | -0,1083 |
| rs2421016 | 0,0382 | 0,0265 | 0,0382 | -0,1582 | 0,0936 |
| rs689 | 7,87E-09 | 0,0564 | -0,2609 | 0,0942 | 0,1103 |
| rs7111341 | 0,0031 | 0,0408 | -0,0849 | -0,0221 | 0,0662 |
| rs231362 | 0,0021 | 0,1099 | -1,60E-04 | 0,0552 | -0,1650 |
| rs231361 | 0,0014 | -0,1300 | 0,0331 | 0,1309 | -0,0340 |
| rs5215 | 0,0030 | -0,0690 | -0,1222 | 0,1203 | 0,0709 |
| rs7944584 | 0,0175 | 0,1601 | 0,0037 | -0,0817 | -0,0821 |

(continued)

| Attribute | P value | Logistic regression coefficient | | | |
|---|---|---|---|---|---|
| | | CONTROL (0) | T1D (1) | T2D (2) | MONOGENIC diabetes(3) |
| | | Intercept $\beta_0 = 0,2999$ | Intercept $\beta_0 = -1,1427$ | Intercept $\beta_0 = 0,3259$ | Intercept $\beta_0 = 0,6069$ |
| rs1552224 | 0,0405 | 0,1596 | 0,0487 | -0,2304 | 0,0220 |
| rs10219509 | 0,0465 | 0,1246 | -0,0753 | 0,0397 | -0,0891 |
| rs7138803 | 0,0027 | 0,0371 | -0,1449 | 0,0545 | 0,0534 |
| rs4759229 | 0,0012 | -0,0648 | 0,1002 | 0,0271 | -0,0624 |
| rs653178 | 0,0057 | 0,0262 | 0,0751 | 0,0310 | -0,1323 |
| rs7957197 | 0,0335 | 0,0944 | 9,43E-04 | -0,0930 | -0,0023 |
| rs9585056 | 0,0037 | -0,0428 | 0,1842 | -0,1611 | 0,0196 |
| rs2289702 | 0,0141 | 0,0132 | -0,0652 | 0,0232 | 0,0289 |
| rs8042680 | 1,62E-04 | -0,1754 | 0,0959 | 0,0011 | 0,0784 |
| rs12899811 | 0,0331 | -0,1208 | -0,0236 | 0,1020 | 0,0426 |
| rs12444268 | 0,0054 | 0,0030 | 0,0374 | 0,0837 | -0,1241 |
| rs9924471 | 0,0074 | -0,0406 | 0,2770 | -0,0891 | -0,1473 |
| rs7202877 | 0,0058 | -0,1257 | 0,0457 | 0,0333 | 0,0467 |
| rs45450798 | 0,0034 | -0,0974 | 0,1005 | 0,0756 | -0,0787 |
| rs763361 | 0,0077 | -0,2562 | 0,1015 | 0,0385 | 0,1162 |
| rs10423928 | 0,0268 | 0,0158 | -0,0196 | 0,1415 | -0,1377 |
| rs5763779 | 0,0039 | -0,0521 | 0,1010 | 0,0260 | -0,0749 |

Table 4: Regression coefficients of selected attributes for T1 D, T2D and monogenic diabetes.

| ATTRIBUTE | P value | LOGISTIC REGRESSION COEFFICIENT | | |
|---|---|---|---|---|
| | | T1D (0) | T2D (1) | MONOGENIC DIABETES (2) |
| | | Intercept $\beta_0 = -1,2618$ | Intercept $\beta_0 = 0,7238$ | Intercept $\beta_0 = 0,5380$ |
| rs10401969 | 1,8E-130 | 0,2033 | 0,3603 | -0,5635 |
| rs36608 | 3,3E-129 | -0,0258 | 0,0308 | -0,0050 |
| rs17791513 | 9,4E-125 | 0,4332 | -0,0713 | -0,3619 |
| rs16996148 | 1E-117 | 0,0696 | -0,3506 | 0,2810 |
| rs429358 | 1,4E-101 | -0,1026 | 0,0409 | 0,0617 |
| rs10503669 | 1,59E-94 | -0,1314 | 0,1573 | -0,0259 |
| rs1801282 | 2,27E-93 | -0,0640 | -0,1859 | 0,2499 |
| rs1552224 | 4,87E-90 | 0,1868 | -0,2542 | 0,0674 |
| rs9470794 | 7,49E-89 | 0,0714 | -0,1411 | 0,0697 |
| rs10203174 | 1,95E-85 | -0,2806 | -0,0501 | 0,3307 |
| rs9921586 | 8,28E-78 | 0,1527 | -0,0151 | -0,1376 |
| rs1531343 | 1,07E-75 | 0,1177 | 0,0212 | -0,1389 |
| rs10885122 | 6,89E-73 | -0,0629 | -0,0228 | 0,0856 |
| rs10923931 | 4,43E-71 | -0,1005 | -0,0445 | 0,1451 |

(continued)

| ATTRIBUTE | P value | LOGISTIC REGRESSION COEFFICIENT | | |
|---|---|---|---|---|
| | | T1D (0) | T2D (1) | MONOGENIC DIABETES (2) |
| | | Intercept $\beta_0 = -1{,}2618$ | Intercept $\beta_0 = 0{,}7238$ | Intercept $\beta_0 = 0{,}5380$ |
| rs7607980 | 1,03E-65 | -0,0345 | 0,0455 | -0,0110 |
| rs1106240 | 7,08E-65 | -0,0333 | -0,0658 | 0,0990 |
| rs17168486 | 3,75E-63 | -0,1253 | 0,1098 | 0,0155 |
| rs1031605 | 1,33E-60 | -0,2974 | 0,2034 | 0,0940 |
| rs35767 | 1,45E-60 | 0,0396 | 0,1634 | -0,2030 |
| rs10842994 | 6,54E-60 | -0,1937 | 0,0258 | 0,1679 |
| rs10219509 | 7,21E-59 | 0,1148 | -0,0322 | -0,0826 |
| rs831571 | 9,25E-58 | 0,1570 | 0,0894 | -0,2464 |
| rs10811661 | 1,37E-55 | 0,1226 | -0,0838 | -0,0389 |
| rs17265513 | 2,2E-55 | -0,1142 | 0,0718 | 0,0423 |
| rs1182397 | 4,97E-54 | 0,0495 | -0,0394 | -0,0101 |
| rs10840595 | 9,18E-54 | -0,0383 | -0,0905 | 0,1287 |
| rs553668 | 1,5E-53 | -0,1353 | 0,0530 | 0,0822 |
| rs11920090 | 1,32E-52 | -0,205 | 0,1847 | 0,0203 |
| rs4812829 | 1,74E-52 | 0,0110 | -0,0626 | 0,0516 |
| rs10146997 | 5,89E-52 | 0,0970 | -0,0752 | -0,0218 |
| rs61839660 | 4,1E-133 | -0,1076 | 0,5564 | -0,4487 |
| rs12189871 | 3,8E-115 | 0,1166 | -0,0610 | -0,0556 |
| rs144530872 | 1,1E-111 | 0,1295 | -0,0574 | -0,0721 |
| rs2524277 | 2,7E-110 | -0,0430 | 0,2263 | -0,1833 |
| rs2289702 | 2,8E-105 | -0,3142 | 0,0687 | 0,2455 |
| rs12251307 | 3,6E-103 | -0,0179 | -0,2380 | 0,2559 |
| rs16956936 | 2,8E-102 | 0,0378 | 0,1223 | -0,1602 |
| rs9259118 | 2,51E-98 | 0,1747 | 0,0893 | -0,264 |
| rs72848653 | 5,46E-96 | 0,3385 | -0,0559 | -0,2827 |
| rs17211699 | 2,36E-88 | 0,4525 | -0,3496 | -0,1029 |
| rs10947332 | 3,03E-87 | 0,3354 | -0,0638 | -0,2716 |
| rs371250843 | 7,94E-78 | -0,0148 | 0,0374 | -0,0226 |
| rs3024505 | 1,39E-76 | 0,0629 | -0,0726 | 0,0097 |
| rs9269173 | 8,33E-76 | 0,2179 | 0,0240 | -0,2419 |
| rs7454108 | 2,23E-73 | 1,5615 | -0,6560 | -0,9055 |
| rs425105 | 5,19E-69 | -0,2953 | 0,0487 | 0,2466 |
| rs45450798 | 6,29E-60 | -0,0592 | -0,0531 | 0,1123 |
| rs2187668 | 1,16E-58 | 1,2050 | -0,7510 | -0,4540 |
| rs11258747 | 2,04E-55 | 0,1925 | -0,2520 | 0,0595 |
| rs2816316 | 3,44E-53 | -0,2071 | 0,2364 | -0,0293 |
| rs947474 | 5,03E-52 | -0,1607 | 0,1532 | 0,0075 |

(continued)

| ATTRIBUTE | P value | LOGISTIC REGRESSION COEFFICIENT | | |
|---|---|---|---|---|
| | | T1D (0) | T2D (1) | MONOGENIC DIABETES (2) |
| | | Intercept $\beta_0$ = - 1,2618 | Intercept $\beta_0$ = 0,7238 | Intercept $\beta_0$ = 0,5380 |
| rs9924471 | 8,29E-52 | 0,0456 | 0,0029 | -0,0485 |
| rs559242105 | 2,44E-51 | 0,1907 | -0,2556 | 0,0648 |
| rs72928038 | 2,79E-51 | 0,0387 | -0,1586 | 0,1198 |
| rs2476601 | 7,31E-51 | 0,6627 | -0,5258 | -0,1368 |
| rs2104286 | 9,76E-51 | -0,1175 | 0,0430 | 0,0745 |
| rs7111341 | 7,72E-44 | -0,1079 | 0,0606 | 0,0473 |
| rs17574546 | 1,88E-43 | 0,0130 | -0,0624 | 0,0494 |
| rs2327832 | 1,88E-40 | -0,0085 | -0,0505 | 0,0589 |
| rs3842753 | 1,93E-39 | -0,1886 | 0,1189 | 0,0696 |

Table 5: Hyperparameters used for multinomial models.

| Multi class | Penalty | C | Solver |
|---|---|---|---|
| Multinomial | l2 | 0,3097 | lbfgs |

*1.4 Results*

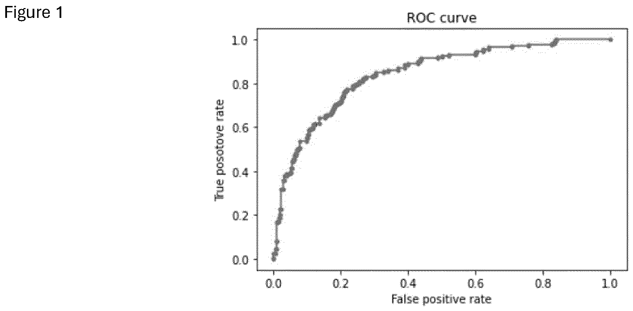

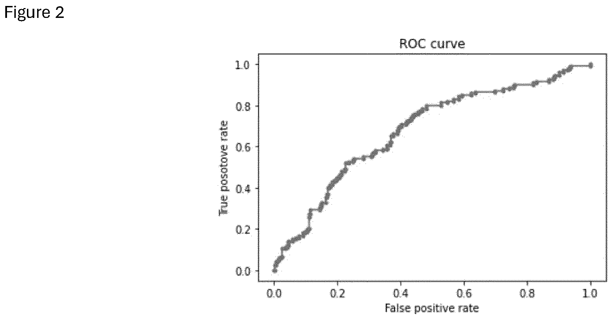

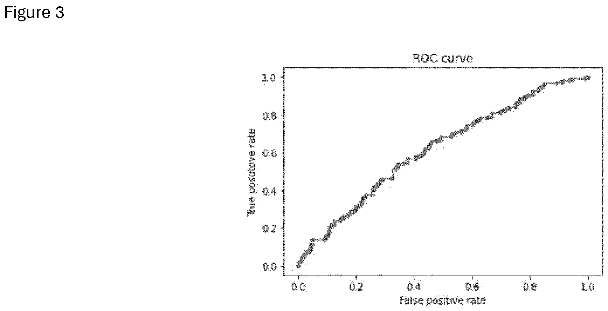

When evaluating the multinomial model developed for the four groups, first, the overall (Area under the curve) AUC was calculated. As mentioned above, in this Multinomial Logistic Regression four probabilities are obtained, one for each group, therefore four AUCs are also obtained (see Table 6 and Figures 1-3). The overall AUC is the average of the four AUCs.

For an algorithm to be identified as a prediction algorithm, it needs to have an (AUC) greater than 0.7. From the overall AUC (Table 7), it can be seen that the multinomial PRS developed for the four classes can therefore be used for diabetes type prediction. Furthermore, the difference between the training AUC and the testing AUC was not very high (Table 7), making it possible to rule out the phenomenon of model overfitting.

Table 6: Area under the curve (AUC) value

| | Control (0) | T1D (1) | T2D (2) | Monogenic Diabetes |
|---|---|---|---|---|
| AUC | 0.69 | 0.84 | 0.68 | 0.62 |

Table 7: AUC values for training and testing data.

| Training AUC | Testing AUC |
|---|---|
| 0.75 | 0.71 |

*EXAMPLE 2*

A risk assessment model to identify individuals at high risk of T1D was developed and validated using a machine learning algorithm. The model can differentiate between those at risk of T1D compared to those not at risk of diabetes or those at risk of T2D or MODY. In this example, only two categories were compared in each model (i.e. T1D vs control, T2D or MODY, or T1D vs T2D, or T1D vs MODY). Therefore the models used were binomial.

*2.1 Cohorts studied*

The cohorts used in this study are the same as those described above in section 1.1.

*2.2 Data processing and attribute selection*

*2.21 Data preprocessing*

The data obtained from Biocruces comprised approximately 14 million SNPs and therefore a smaller selection of 306 SNPs was made. These 306 SNPs included SNPs associated with T1D and T2D. 25 clinical variables were also examined.

When preparing the data for the subsequent steps, it was first ensured that the classes were balanced and that there were no missing values. SNPs could have a value of 0, 1 or 2, depending on the number of different alleles with respect to the reference genome. Any missing values were identified and replaced by the most common value for categorical attributes.

*2.22 Attribute selection*

Fisher's Exact Test (Fisher's Exact Test) and FDR correction (FDR Correction) were carried out.

Finally, the Pearson correlation test was carried out on these selections. This entire process has been carried out with the ScyPy and Sklearn libraries, and the final selection of these models can be seen in Tables 8 and 10.

*2.3 Selection of machine learning models and their hyperparameters*

Logistic Regression was chosen for these models. Both the training of the algorithm and the selection of the hyperparameters were carried out with the Sklearn library, and both the hyperparameters selected for each algorithm as well as the coefficients that they have assigned to the attributes are detailed in Tables 8-11.

Table 8 shows the attributes selected for the machine learning model developed to predict the chance of developing T1D vs control, T2D or monogenic diabetes, along with their regression coefficients.

Table 8: attributes selected for the machine learning model developed to predict the chance of developing T1D vs control, T2D or monogenic diabetes, along with their regression coefficients.

| ATTRIBUTE | P VALUE | ODDS RATIO | LOGISTIC REGRESSION COEFFICIENT |
|---|---|---|---|
| | | | Intercept $\beta0$ = -1,6756 |
| **rs2187668** | 9,49E-43 | 6,8836 | 1,5077 |
| **rs7454108** | 2,10E-37 | 6,3563 | 1,8887 |
| **rs9269173** | 3,07E-27 | 5,3244 | 0,6369 |
| **rs9268645** | 2,65E-11 | 3,2062 | 0,1130 |
| **rs371250843** | 3,49E-09 | 2,7405 | 0,2766 |
| **rs3842753** | 5,28E-06 | 0,5032 | -0,4489 |
| **rs2476601** | 3,06E-05 | 2,4052 | 0,9463 |
| **rs17211699** | 4,12E-05 | 0,4609 | 0,5182 |
| **rs559242105** | 0,0012 | 1,7687 | 0,1225 |
| **rs10947332** | 0,0011 | 0,5257 | 0,4282 |
| **rs9924471** | 0,0151 | 1,5839 | 0,3269 |
| **rs4788084** | 0,0386 | 0,6661 | -0,4502 |
| **rs2524277** | 0,0404 | 0,5779 | -0,0807 |

Table 9: hyperparameters used for the machine learning model (T1D vs control, T2D or monogenic diabetes).

| *Penalty* | *C* | *Solver* |
|---|---|---|
| l2 | 0,1826 | newton-cg |

Table 10: attributes selected for the machine learning model developed to predict the chance of developing T1D vs monogenic diabetes, along with their regression coefficients.

| ATTRIBUTE | P VALUE | ODDS RATIO | LOGISTIC REGRESSION COEFFICIENT |
|---|---|---|---|
| | | | Intercept $\beta_0$= -2,3739 |
| rs7454108 | 5,41E-37 | 6,4895 | 1,7206 |
| rs9273369 | 3,39E-37 | 5,9967 | 1,4993 |
| rs9269173 | 1,17E-24 | 4,8755 | 0,1360 |
| rs9268645 | 3,91E-14 | 3,6474 | 0,1226 |
| rs371250843 | 3,30E-07 | 2,4447 | 0,1882 |
| rs3842753 | 7,30E-05 | 0,5307 | -0,3847 |
| rs17211699 | 8,18E-05 | 0,4636 | -0,1449 |
| rs2476601 | 0.0010 | 2,0833 | 0,3120 |
| rs4607517 | 0.0024 | 0,5862 | -0,1597 |
| rs4759229 | 0.0043 | 1,6619 | 0,3222 |
| rs10947332 | 0.0091 | 0,5642 | 0,3271 |
| rs3184504 | 0.0133 | 1,6260 | 0,3398 |
| rs5763779 | 0.0126 | 1,5855 | 0,1014 |
| rs559242105 | 0.0184 | 1,5703 | 0,1239 |
| rs7111341 | 0.0200 | 0,6499 | 0,0617 |
| rs831571 | 0.0333 | 1,5423 | 0,4125 |
| rs9924471 | 0.0353 | 1,5132 | 0,1162 |
| rs2289702 | 0.0342 | 0,5916 | -0,3939 |

Table 11: hyperparameters used for the machine learning model (T1D vs monogenic diabetes).

| *Penalty* | *C* | *Solver* |
|---|---|---|
| l2 | 0,1186 | newton-cg |

### 2.4 Results

The machine learning models were able to distinguish susceptibility to T1D from T2D, MODY or no diabetes (controls) with exceptional accuracy as shown in Table 12 below.

Table 12: AUC for the binomial machine learning models.

| Comparison | AUC training set | AUC validation set | Sensitivity | Specificity |
|---|---|---|---|---|
| **T1D vs [Control, MODY, T2D]** | **0.87** | **0.87** | **0.81** | **0.83** |
| T1D vs MODY | 0.89 | 0.86 | 0.80 | 0.80 |
| T1D vsT2D | 0.88 | 0.86 | 0.81 | 0.78 |

Overall the machine learning models can differentiate susceptibility to T1D from susceptibility to T2D or MODY, or no susceptibility.

### References

A number of publications are cited above in order to more fully describe and disclose the invention and the state of the art to which the invention pertains. Full citations for these references are provided below. The entirety of each of these references is incorporated herein.

Alonso GT, Coakley A, Pyle L, Manseau K, Thomas S, Rewers A. Diabetic ketoacidosis at diagnosis of type 1 diabetes in Colorado children, 2010-2017. Diabetes Care 2020;43:117-121

American Diabetes Association, "What is Diabetes", https://ada.com/conditions/diabetes/, accessed 3 June 2024.

DiMeglio LA, Evans-Molina C, Oram RA. Type 1 diabetes. Lancet. 2018 Jun 16;391(10138):2449-2462. doi: 10.1016/80140-6736(18)31320-5. PMID: 29916386; PMCID: PMC6661119.

Gregory GA, Robinson TIG, Linklater SE, Wang F, Colagiuri S, de Beaufort C, Donaghue KC; International Diabetes Federation Diabetes Atlas Type 1 Diabetes in Adults Special Interest Group; Magliano DJ, Maniam J, Orchard TJ, Rai P, Ogle GD. Global incidence, prevalence, and mortality of type 1 diabetes in 2021 with projection to 2040: a modelling study. Lancet Diabetes Endocrinol. 2022 Oct;10(10):741-760. doi: 10.1016/S2213-8587(22)00218-2. Epub 2022 Sep 13. Erratum in: Lancet Diabetes Endocrinol. 2022 Oct 7;: PMID: 36113507.

Goyal R, Singhal M, Jialal I. Type 2 Diabetes. [Updated 2023 Jun 23]. In: StatPearls [Internet]. Treasure Island (FL): StatPearls Publishing; 2024 Jan-. Available from: https://www.ncbi.nlm.nih.gov/books/NBK513253/

Herold KC, Bundy BN, Long SA, et al..; Type 1 Diabetes TrialNet Study Group . An anti-CD3 antibody, teplizumab, in relatives at risk for type 1 diabetes. N Engl J Med 2019;381:603-613

IDF Diabetes Atlas, "Diabetes around the world in 2021", https://diabetesatlas.org/, accessed 3 June 2024.

Katsarou A, Gudbjörnsdottir S, Rawshani A, Dabelea D, Bonifacio E, Anderson BJ, Jacobsen LM, Schatz DA, Lernmark Å. Type 1 diabetes mellitus. Nat Rev Dis Primers. 2017 Mar 30;3:17016. doi: 10.1038/nrdp.2017.16. PMID: 28358037.

Michelle So, Cate Speake, Andrea K Steck, Markus Lundgren, Peter G Colman, Jerry P Palmer, Kevan C Herold, Carla J Greenbaum, Advances in Type 1 Diabetes Prediction Using Islet Autoantibodies: Beyond a Simple Count, Endocrine Reviews, Volume 42, Issue 5, October 2021, Pages 584-604.

Primavera M, Giannini C, Chiarelli F. Prediction and Prevention of Type 1 Diabetes. Front Endocrinol (Lausanne). 2020 Jun 2;11:248. doi: 10.3389/fendo.2020.00248. PMID: 32670194; PMCID: PMC7326081.

Sims EK, Besser REJ, Dayan C, Geno Rasmussen C, Greenbaum C, Griffin KJ, Hagopian W, Knip M, Long AE, Martin F, Mathieu C, Rewers M, Steck AK, Wentworth JM, Rich SS, Kordonouri O, Ziegler AG, Herold KC; NIDDK Type 1 Diabetes TrialNet Study Group. Screening for Type 1 Diabetes in the General Population: A Status Report and Perspective. Diabetes. 2022 Apr 1;71(4):610-623. doi: 10.2337/dbi20-0054. PMID: 35316839; PMCID: PMC9114719.

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

## Claims

1.   A method of predicting diabetes status in a subject, the method comprising determining the identity of at least one allele at each of at least four positions of single nucleotide polymorphism (SNP) selected from:

     rs371250843;
     rs9269173;
     rs17211699;
     rs2476601;
     rs10947332;
     rs7454108;
     rs9924471; and
     rs559242105,

     and/or an SNP in linkage disequilibrium with any one of said SNPs at $r^2 > 0.8$.

2.   The method of claim 1 wherein the method further comprises determining the identity of at least one allele of at least one of the SNPs selected from:

     rs2524277;
     rs9268645;
     rs2187668;
     rs3842753; and
     rs4788084,

and/or an SNP in linkage disequilibrium with any one of said SNPs at $r^2 > 0.8$.

3.  The method of any one of claims 1 or 2 wherein the predictive allele at each SNP comprises:

    Del G at rs371250843;
    A at rs9269173;
    T at rs17211699;
    G at rs2476601;
    A at rs10947332;
    C at rs7454108;
    A at rs9924471;
    dupTA at rs559242105;
    A at rs2524277;
    G at rs9268645;
    T at rs2187668;
    T at rs3842753; and
    T at rs4788084.

4.  The method according to any one of the preceding claims, wherein allele determination is carried out at not more than 75 SNP positions.

5.  The method according to any one of the preceding claims, wherein the method comprises determining the identity of both alleles at each SNP thereby obtaining the genotype of the subject at each SNP.

6.  The method according to any one of the preceding claims, wherein the method comprises assaying a DNA-containing sample that has previously been obtained from said subject.

7.  The method according to claim 6, wherein said sample is selected from the group consisting of: blood, hair, skin, amniotic fluid, buccal swab, saliva, and faeces.

8.  The method according to claim 6 or claim 7, wherein the method comprises isolating and/or amplifying genomic DNA from said subject.

9.  The method according to any one of the preceding claims, wherein determining the identity of said at least one allele at each SNP comprises: probe hybridization, real time PCR, array analysis, bead analysis, primer extension, restriction analysis and/or DNA sequencing.

10. The method of any one of the preceding claims wherein predicting diabetes status comprises classifying the subject into one of the groups selected from susceptibility to T1D, susceptibility to T2D, susceptibility to monogenic diabetes and no susceptibility to diabetes.

11. The method of any one of claims 1-9 wherein predicting diabetes status comprises classifying the subject into one of the groups selected from susceptibility to T1D, susceptibility to T2D and susceptibility to monogenic diabetes.

12. The method of claim 10 or claim 11 wherein the method comprises determining the identity of the genotype of the subject at each SNP and inputting the genotype identities into a probability function to compute said classification.

13. The method of claim 12 wherein the probability function comprises a logistic regression algorithm.

14. The method of any one of claims 1-9 wherein predicting diabetes status comprises assessing the probability that a subject will develop T1D compared to T2D, monogenic diabetes and/or no diabetes.

15. The method of claim 14 wherein predicting diabetes status comprises assessing the probability that a subject will develop T1D compared to T2D, monogenic diabetes and no diabetes.

16. The method of claim 14 wherein predicting diabetes status comprises assessing the probability that a subject will develop T1D compared to T2D.

17. The method of claim 14 wherein predicting diabetes status comprises assessing the probability that a subject will develop T1D compared to monogenic diabetes.

18. The method of any one of claims 14-17 wherein the method comprises determining the identity of the genotype of the subject at each SNP, and wherein the method further comprises computing a T1D risk score for said subject based on the identity of the genotype at each SNP.

19. The method of claim 18 wherein the method comprises inputting the genotype identities into a probability function to compute said risk score.

20. The method of claim 19 wherein the probability function comprises a logistic regression algorithm.

21. The method according to any one of claims 18-20 wherein computing the risk score from the genotype data comprises weighting the contribution of each SNP found to be present such that the contribution of each SNP to the risk score is commensurate with an odds ratio for the association of the SNP to type 1 diabetes, as set forth in Tables 8 and 10.

22. The method according to any one of claims 18-21 wherein computing said risk score comprises using the following formula:

$$risk\ score\ \ p = \frac{1}{1 + e^{-(-\beta_0 + \sum_{i=1}^{m} \beta_i x_i)}}$$

wherein $\beta_0$ is an intercept weight, $\beta$ is a vector of weights for each of m variables and x is a vector of variables associated with the subject, wherein the variables comprise the number of predictive alleles present at each SNP and optionally variables derived therefrom.

23. A system comprising:

   a). a processor; and
   b). a computer readable medium comprising instructions that, when executed by the processor, cause the processor to perform the method of any one of claims 12 to 13 or 18-22.

24. A non-transitory computer readable medium or media comprising instructions that, when executed by at least one processor, cause the at least one processor to perform the method of any one of claims 12-13 or 19-23.

25. The method according to any one of claims 14-22, wherein following identification of the at least one allele at each single nucleotide polymorphism (SNP), if the subject is determined to be at risk of developing Type 1 Diabetes, the method further comprises administering to the subject a test selected from the group consisting of: detection of type 1 diabetes autoantibodies, a glycated hemoglobin (A1C) test, a blood sugar test or a C-peptide test.

26. The method of claim 25 wherein the detection of type 1 diabetes autoantibodies comprises detection of islet cell autoantibodies, glutamic acid decarboxylase autoantibodies, insulin autoantibodies and/or protein tyrosine phosphatase autoantibodies.

27. A method of preventing or delaying onset of Type 1 Diabetes in a subject, the method comprising determining the identity of at least one allele at each of at least four positions of single nucleotide polymorphism (SNP) selected from the following:

   rs371250843;
   rs9269173;
   rs17211699;
   rs2476601;
   rs10947332;
   rs7454108;
   rs9924471; and
   rs559242105,

and/or an SNP in linkage disequilibrium with any one of said SNPs at $r^2 > 0.8$;

determining the subject to be at risk of developing T1D, and administering a medicament for preventing or delaying the onset of T1D.

28. The method according to claim 27 wherein the method further comprises determining the identity of at least one allele of at least one of the SNPs selected from:

rs2524277;
rs9268645;
rs2187668;
rs3842753; and
rs4788084

and/or an SNP in linkage disequilibrium with any one of said SNPs at $r^2 > 0.8$.

29. The method according to claim 27 or claim 28 wherein the medicament is Teplizumab.

30. A genotyping tool for use in a method of any one of claims 1 to 22 or 25 to 29, said tool comprising an array having a plurality of oligonucleotide probe pairs, each of said probe pairs comprising a first probe specific for a first allele of a single nucleotide polymorphism (SNP) and a second probe specific for a second allele of the SNP, wherein said plurality of oligonucleotide probe pairs comprises probe pairs that interrogate at least four of the following SNPs:

rs371250843;
rs9269173;
rs17211699;
rs2476601;
rs10947332;
rs7454108;
rs9924471; and
rs559242105,

and/or an SNP in linkage disequilibrium with any one of said SNPs at $r^2 > 0.8$.

31. The genotyping tool according to claim 30 further comprising oligonucleotide probe pairs that interrogate at least one of the SNPs selected from:

rs2524277;
rs9268645;
rs2187668;
rs3842753; and
rs4788084,

and/or an SNP in linkage disequilibrium with any one of said SNPs at $r^2 > 0.8$.

32. The genotyping according to claim 30, wherein the oligonucleotide probes of the array that interrogate SNPs selected from rs371250843, rs9269173, rs17211699, rs2476601, rs10947332, rs7454108, rs9924471 and rs559242105 make up at least 50% of the probes present in the array.

33. The genotyping tool according to claims 30-32 wherein the total number of different SNPs for which allele-specific probes are provided does not exceed 25.

34. The genotyping tool according to any one of claims 30-33, wherein the tool is in the form of a TaqMan® OpenArray® SNP genotyping platform, a Dynamic Array integrated fluidic circuits (IFC) genotyping platform, a next-generation sequencing system, or a MassARRAY® system.

35. The genotyping tool according to any one of claims 30-34, wherein the allele-specific oligonucleotide probes are each covalently attached to a fluorophore.

36. The genotyping tool according to any one of claims 30-35, wherein the tool further comprises a primer pair for each of said SNPs, said primer pair for each SNP comprising an oligonucleotide primer that hybridizes to a target sequence upstream of the SNP and an oligonucleotide primer that hybridizes to a target sequence downstream of the SNP.

37. The genotyping tool according to any one of claims 30-36, wherein the tool further comprises one or more reagents for amplification of DNA comprising said SNPs and/or for detection of said allele-specific probes.

38. A diabetes classification system for use in a method as defined in any one of claims 1-10 or 12-13, the system comprising a genotyping tool as defined in any one of claims 30-37 and a computer programmed to classify a subject into one of the groups selected from susceptibility to T1D, susceptibility to T2D, susceptibility to monogenic diabetes and no susceptibility to diabetes from the genotype data of the subject at each of the SNPs set forth in claim 1.

39. A diabetes classification system for use in a method as defined in any one of claims 1-9 or 11-13, the system comprising a genotyping tool as defined in any one of claims 30-37 and a computer programmed to classify a subject into one of the groups selected from susceptibility to T1D, susceptibility to T2D and susceptibility to monogenic diabetes from the genotype data of the subject at each of the SNPs set forth in claim 1.

40. A Type 1 Diabetes risk assessment system for use in a method as defined in any one of claims 1-9 or 14-22, the system comprising a genotyping tool as defined in any one of claims 30-37 and a computer programmed to compute a Type 1 Diabetes risk score from the genotype data of the subject at each of at the SNPs set forth in claim 1.

41. The Type 1 Diabetes risk assessment system of claim 40, wherein computing the risk score from the genotype data comprises weighting the contribution of each SNP found to be present such that the contribution of each SNP to the risk score is commensurate with an odds ratio for the association of the SNP to type 1 diabetes, as set forth in Tables 8 or 10.

Figure 1

ROC curve

Figure 2

ROC curve

Figure 3

ROC curve

Europäisches
Patentamt

European
Patent Office

Office européen
des brevets

# EUROPEAN SEARCH REPORT

**Application Number**

EP 24 38 2651

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | RICHARD A. ORAM ET AL: "A Type 1 Diabetes Genetic Risk Score Can Aid Discrimination Between Type 1 and Type 2 Diabetes in Young Adults", DIABETES CARE, vol. 39, no. 3, 17 November 2015 (2015-11-17), pages 337-344, XP055408771, US ISSN: 0149-5992, DOI: 10.2337/dc15-1111 * the whole document * | 1-15 | INV. C12Q1/6883 |
| Y | REDONDO MARIA J ET AL: "Genetic Risk Scores for Type 1 Diabetes Prediction and Diagnosis", CURRENT DIABETES REPORTS, CURRENT SCIENCE, PHILADELPHIA, VA, US, vol. 17, no. 12, 28 October 2017 (2017-10-28), pages 1-10, XP036374561, ISSN: 1534-4827, DOI: 10.1007/S11892-017-0961-5 [retrieved on 2017-10-28] * the whole document * | 1-15 | |
| Y | WO 2019/002364 A1 (HELMHOLTZ ZENTRUM MUENCHEN DEUTSCHES FORSCHUNGSZENTRUM GESUNDHEIT & UM) 3 January 2019 (2019-01-03) * the whole document * | 1-15 | TECHNICAL FIELDS SEARCHED (IPC)  C12Q |
| Y | US 2012/309639 A1 (HAKONARSON HAKON [US] ET AL) 6 December 2012 (2012-12-06) * the whole document * | 1-15 | |

-/--

The present search report has been drawn up for all claims

| Place of search | Date of completion of the search | Examiner |
|---|---|---|
| The Hague | 4 February 2025 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

## EUROPEAN SEARCH REPORT

**Application Number**

EP 24 38 2651

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ANDERSEN METTE K: "New Insights into the Genetics of Latent Autoimmune Diabetes in Adults", CURRENT DIABETES REPORTS, CURRENT SCIENCE, PHILADELPHIA, VA, US, vol. 20, no. 9, 28 July 2020 (2020-07-28), XP037205105, ISSN: 1534-4827, DOI: 10.1007/S11892-020-01330-Y [retrieved on 2020-07-28] * the whole document * ----- | 1-15 | |
| Y | RAJASHREE MISHRA ET AL: "Relative contribution of type 1 and type 2 diabetes loci to the genetic etiology of adult-onset, non-insulin-requiring autoimmune diabetes", BMC MEDICINE, vol. 15, no. 88, 25 April 2017 (2017-04-25), pages 1-10, XP055549940, DOI: 10.1186/s12916-017-0846-0 * the whole document * ----- | 1-15 | TECHNICAL FIELDS SEARCHED (IPC) |
| Y | CN 117 778 564 A (THE SECOND XIANGYA HOSPITAL OF CENTRAL SOUTH UNIV) 29 March 2024 (2024-03-29) * the whole document * ----- -/-- | 1-15 | |

The present search report has been drawn up for all claims

| Place of search | Date of completion of the search | Examiner |
|---|---|---|
| The Hague | 4 February 2025 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 24 38 2651

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Anonymous Carolyn ET AL: "Genetic discovery and risk prediction for type 1 diabetes in individuals without high-risk HLA-DR3/DR4 haplotypes - PMC", medRxiv, 4 December 2023 (2023-12-04), pages 1-25, XP093246470, DOI: 10.1101/2023.11.11.23298405 Retrieved from the Internet: URL:https://pmc.ncbi.nlm.nih.gov/articles/PMC10659516/ * the whole document * | 1-15 | |

-----

TECHNICAL FIELDS
SEARCHED    (IPC)

The present search report has been drawn up for all claims

| Place of search | Date of completion of the search | Examiner |
|---|---|---|
| The Hague | 4 February 2025 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................
& : member of the same patent family, corresponding document

EPO FORM 1503 03.82 (P04C01)

page 3 of 3

## ANNEX TO THE EUROPEAN SEARCH REPORT
## ON EUROPEAN PATENT APPLICATION NO.

EP 24 38 2651

This annex lists the patent family members relating to the patent documents cited in the above-mentioned European search report.
The members are as contained in the European Patent Office EDP file on
The European Patent Office is in no way liable for these particulars which are merely given for the purpose of information.

04-02-2025

| Patent document cited in search report | | Publication date | Patent family member(s) | | Publication date |
|---|---|---|---|---|---|
| WO 2019002364 | A1 | 03-01-2019 | CA | 3067713 A1 | 03-01-2019 |
| | | | CN | 111065749 A | 24-04-2020 |
| | | | EP | 3645736 A1 | 06-05-2020 |
| | | | US | 2020126671 A1 | 23-04-2020 |
| | | | WO | 2019002364 A1 | 03-01-2019 |
| US 2012309639 | A1 | 06-12-2012 | CA | 2776588 A1 | 14-04-2011 |
| | | | EP | 2486402 A1 | 15-08-2012 |
| | | | US | 2012309639 A1 | 06-12-2012 |
| | | | WO | 2011044458 A1 | 14-04-2011 |
| CN 117778564 | A | 29-03-2024 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CUTLER DJ** ; **ZWICK ME** ; **CARRASQUILLO MN** ; **YOHN CT** ; **TOBI KP** ; **KASHUK C** ; **MATHEWS DJ** ; **SHAH N** ; **EICHLER EE** ; **WARRINGTON JA**. *Genome Research*, 2001, vol. 11, 1913-1925
- **ALONSO GT** ; **COAKLEY A** ; **PYLE L** ; **MANSEAU K** ; **THOMAS S** ; **REWERS A**. Diabetic ketoacidosis at diagnosis of type 1 diabetes in Colorado children, 2010-2017. *Diabetes Care*, 2020, vol. 43, 117-121
- What is Diabetes. *American Diabetes Association*, 03 June 2024, https://ada.com/conditions/diabetes
- **DIMEGLIO LA** ; **EVANS-MOLINA C** ; **ORAM RA**. Type 1 diabetes. *Lancet*, 16 June 2018, vol. 391 (10138), 2449-2462
- **GREGORY GA** ; **ROBINSON TIG** ; **LINKLATER SE** ; **WANG F** ; **COLAGIURI S** ; **DE BEAUFORT C** ; **DONAGHUE KC**. *International Diabetes Federation Diabetes Atlas Type 1 Diabetes in Adults Special Interest Group*
- **MAGLIANO DJ** ; **MANIAM J** ; **ORCHARD TJ** ; **RAI P** ; **OGLE GD**. Global incidence, prevalence, and mortality of type 1 diabetes in 2021 with projection to 2040: a modelling study. *Lancet Diabetes Endocrinol.*, October 2022, vol. 10 (10), 741-760
- **HEROLD KC** ; **BUNDY BN** ; **LONG SA et al.** Type 1 Diabetes TrialNet Study Group . An anti-CD3 antibody, teplizumab, in relatives at risk for type 1 diabetes.. *N Engl J Med*, 2019, vol. 381, 603-613
- Diabetes around the world in 2021. *IDF Diabetes Atlas*, 03 June 2024, https://diabetesatlas.org
- **KATSAROU A** ; **GUDBJÖRNSDOTTIR S** ; **RAWSHANI A** ; **DABELEA D** ; **BONIFACIO E** ; **ANDERSON BJ** ; **JACOBSEN LM** ; **SCHATZ DA** ; **LERNMARK Å**. Type 1 diabetes mellitus.. *Nat Rev Dis Primers.*, 30 March 2017, vol. 3, 17016
- **MICHELLE SO** ; **CATE SPEAKE** ; **ANDREA K STECK** ; **MARKUS LUNDGREN** ; **PETER G COLMAN** ; **JERRY P PALMER** ; **KEVAN C HEROLD** ; **CARLA J GREENBAUM**. Advances in Type 1 Diabetes Prediction Using Islet Autoantibodies: Beyond a Simple Count. *Endocrine Reviews*, October 2021, vol. 42 (5), 584-604
- **PRIMAVERA M** ; **GIANNINI C** ; **CHIARELLI F**. Prediction and Prevention of Type 1 Diabetes.. *Front Endocrinol (Lausanne)*, 02 June 2020, vol. 11, 248
- **SIMS EK** ; **BESSER REJ** ; **DAYAN C** ; **GENO RASMUSSEN C** ; **GREENBAUM C** ; **GRIFFIN KJ** ; **HAGOPIAN W** ; **KNIP M** ; **LONG AE** ; **MARTIN F**. NIDDK Type 1 Diabetes TrialNet Study Group. Screening for Type 1 Diabetes in the General Population: A Status Report and Perspective. *Diabetes*, 01 April 2022, vol. 71 (4), 610-623
- **SAMBROOK, J.** ; **RUSSEL, D.W.** Molecular Cloning, A Laboratory Manual.. Cold Spring Harbor Laboratory Press, 2001